# EUROPÄISCHE PATENTANMELDUNG

(11) **EP 4 194 582 A1**
(43) Veröffentlichungstag der Anmeldung: **14.06.2023**
(21) Anmeldenummer: 22206885.0
(22) Anmeldetag: 11.11.2022
(51) Int. Cl.: C22F 1/00, A61F 2/24, A61F 2/82, C22F 1/10

(54) **VERFAHREN ZUR FORMGEBUNG EINES FORMGEDÄCHTNISWERKSTÜCKS UND FORMGEBUNGSWERKZEUG ZUR FORMGEBUNG EINES FORMGEDÄCHTNISWERKSTÜCKS**

(30) Priorität: 08.12.2021 DE 102021006050
(71) Anmelder: ADMEDES GmbH, 75179 Pforzheim (DE)
(72) Erfinder: ZENDE, Stefan, 76137 Karlsruhe (DE); HEGEL, Alexander, 76187 Karlsruhe (DE)
(74) Vertreter: Müller-Boré & Partner Patentanwälte PartG mbB

(57) **Zusammenfassung**

Die vorliegende Erfindung betrifft ein Verfahren zur Formgebung eines Formgedächtniswerkstücks und ein Formgebungswerkzeug zur Formgebung eines Formgedächtniswerkstücks, wobei das Verfahren insbesondere die folgenden Schritte aufweist:
- Bereitstellen eines Formgedächtniswerkstücks aufweisend einen ersten Durchmesser und eine vorbestimmte Formgebungstemperatur;
- Anordnen des Formgedächtniswerkstücks an einem Formgebungswerkzeug;
- Erwärmen des Formgedächtniswerkstücks auf die Formgebungstemperatur;
- erstes Aufweiten des Formgedächtniswerkstücks auf einen zweiten Durchmesser, der größer ist als der erste Durchmesser;
- erstes Ändern der Temperatur des Formgedächtniswerkstücks auf eine Zwischentemperatur unterhalb oder oberhalb der Formgebungstemperatur;
- erneutes Bringen des Formgedächtniswerkstücks auf die Formgebungstemperatur;
- zweites Aufweiten des Formgedächtniswerkstücks auf einen dritten Durchmesser, der größer ist als der zweite Durchmesser;
- Abwerfen des Formgedächtniswerkstücks von dem Formgebungswerkzeug;
und
-finales Abkühlen des Formgedächtniswerkstücks auf eine Abkühltemperatur unterhalb der Zwischentemperatur.

Ferner wird ein Formgebungswerkzeug zur Formgebung eines Formgedächtniswerkstücks bereitgestellt.

## Beschreibung

Die vorliegende Erfindung betrifft ein Verfahren zur Formgebung eines Formgedächtniswerkstücks und ein Formgebungswerkzeug zur Formgebung eines Formgedächtniswerkstücks.

Formgedächtnislegierungen und damit hergestellte Formgedächtniswerkstücke sind in verschiedenen Anwendungsszenarien, wie beispielsweise in der Medizintechnik, bekannt. Formgedächtniswerkstücke weisen je nach Umgebungsbedingungen verschiedene Zustände auf, und zeichnen sich dadurch aus, dass bereits bei geringen Änderungen der Umgebungsbedingungen erhebliche Änderungen der Form bzw. der Geometrie des Formgedächtniswerkstücks stattfinden können, in dem sich das Formgedächtniswerkstück an eine bestimmte Form bzw. einen bestimmten Zustand "erinnert". Die charakteristischen Eigenschaften eines Formgedächtniswerkstücks können grundsätzlich in temperaturbedingte Formgedächtniseigenschaften und spannungsbedingte Formgedächtniseigenschaften unterteilt werden, wobei die spannungsbedingten Formgedächtniseigenschaften auch mit dem Begriff der Superelastizität bzw. der superelastischen Eigenschaften beschrieben werden. Die temperaturbedingten Formgedächtniseigenschaften sowie die superelastischen Eigenschaften und die damit verbundenen Zustände eines Formgedächtniswerkstücks, wie insbesondere Geometrie sowie Temperatur und/oder Spannung, bei der die Geometrie eingenommen wird, werden dem Formgedächtniswerkstück dabei im Zuge seiner Fertigung eingeprägt.

Zur Formgebung von Formgedächtniswerkstücken, wie beispielsweise bei Stents und Herzklappenrahmen, und der damit verbundenen Einprägung der temperaturbedingten Formgedächtniseigenschaften und/oder der superelastischen Eigenschaften, sind verschiedene Verfahren bekannt. Im Stand der Technik wird ein Verfahren vorgeschlagen, bei dem ein Formgedächtniswerkstück mit einer Vielzahl von Schritten schrittweise aufgeweitet wird, wobei das Aufweiten selbst bei einer Temperatur um die Raumtemperatur herum stattfindet, und wobei das Formgedächtniswerkstück zwischen den einzelnen Schritten des Aufweitens erwärmt wird. Demgegenüber schlägt das Dokument EP 2 756 109 B1 als weiterer Stand der Technik ein Verfahren zur Formgebung eines Formgedächtniswerkstücks vor, wobei ein Formgedächtniswerkstück zunächst auf eine Formgebungstemperatur erwärmt wird, um im Anschluss in einem einzigen Schritt die Aufweitung des Formgedächtniswerkstücks vorzunehmen, bei dem der ursprüngliche Durchmesser des Formgedächtniswerkstücks um das mindestens zweifache, bis hin zum sechsfachen, vergrößert wird. Danach wird das Formgedächtniswerkstück, zusammen mit dem Formgebungswerkzeug, an dem es zur Aufweitung angeordnet ist, abgekühlt, um somit dem Formgedächtniswerkstück ein gewünschtes Formgedächtnisverhalten einzuprägen. Das Dokument EP 2 756 109 B1 stellt die beiden voranstehend umrissenen Verfahren in den Figuren 1 und 2 des Dokuments EP 2 756 109 B1 gegenüber.

Bei der Formgebung von Formgedächtniswerkstücken treten jedoch verschiedene Problemstellungen auf. So können bei der Verformung Schädigungen am Formgedächtniswerkstück auftreten, welche ein zuverlässiges Einprägen der Formgedächtniseigenschaften verhindern oder erschweren. Beispielhaft können Schädigungen durch lokal überhöhte Dehnungen am Formgedächtniswerkstück verursacht werden. Darüber hinaus ist eine genaue Prozessführung bzgl. der Umformung bzw. Aufweitung eines Formgedächtniswerkstücks und der dabei aufgebrachten Umgebungsbedingungen insbesondere energetisch sehr aufwendig.

Es ist daher Aufgabe der vorliegenden Erfindung ein insbesondere gegenüber dem Dokument EP 2 756 109 B1 verbessertes Verfahren zur Formgebung eines Formgedächtniswerkstücks bereitzustellen. Darüber hinaus ist es Aufgabe der vorliegenden Erfindung ein Formgebungswerkzeug zur verbesserten Formgebung eines Formgedächtniswerkstücks bereitzustellen.

Die vorbeschriebene Aufgabe wird durch die Gegenstände der unabhängigen Ansprüche gelöst, bevorzugte Ausführungsformen sind Gegenstand der abhängigen Ansprüche.

Ein Aspekt der Erfindung betrifft ein Verfahren zur Formgebung eines Formgedächtniswerkstücks, umfassend:
- Bereitstellen eines Formgedächtniswerkstücks aufweisend einen ersten Durchmesser und eine vorbestimmte Formgebungstemperatur;
- Anordnen des Formgedächtniswerkstücks an einem Formgebungswerkzeug;
- Erwärmen des Formgedächtniswerkstücks auf die Formgebungstemperatur;
- erstes Aufweiten des Formgedächtniswerkstücks auf einen zweiten Durchmesser, der größer ist als der erste Durchmesser;
- erstes Ändern der Temperatur des Formgedächtniswerkstücks auf eine Zwischentemperatur unterhalb oder oberhalb der Formgebungstemperatur;
- erneutes Bringen des Formgedächtniswerkstücks auf die Formgebungstemperatur;
- zweites Aufweiten des Formgedächtniswerkstücks auf einen dritten Durchmesser, der größer ist als der zweite Durchmesser;
- Abwerfen des Formgedächtniswerkstücks von dem Formgebungswerkzeug; und
- finales Abkühlen des Formgedächtniswerkstücks auf eine Abkühltemperatur unterhalb der Zwischentemperatur.

Mit anderen Worten betrifft der oben genannte Aspekt ein Verfahren zur Formgebung eines Formgedächtniswerkstücks, wobei insbesondere das Formgedächtniswerkstück zunächst auf die Formgedächtnistemperatur des Formgedächtniswerkstücks erwärmt wird, und nach einem ersten Aufweiten das Formgedächtniswerkstück gegenüber der Formgedächtnistemperatur abgekühlt oder erwärmt wird. Falls das Formgedächtniswerkstück nach dem ersten Aufweiten auf eine Zwischentemperatur abgekühlt wird, nimmt das Formgedächtniswerkstück eine Zwischentemperatur ein, welche niedriger ist als die Formgedächtnistemperatur, auf die das Formgedächtniswerkstück ursprünglich erwärmt wurde, und damit insbesondere verschieden ist von der Formgedächtnistemperatur, auf die das Formgedächtniswerkstück ursprünglich erwärmt wurde. Falls das Formgedächtniswerkstück nach dem ersten Aufweiten erwärmt wird, nimmt das Formgedächtniswerkstück eine Zwischentemperatur ein, welche höher ist als die Formgedächtnistemperatur, auf die das Formgedächtniswerkstück ursprünglich erwärmt wurde, und damit insbesondere verschieden ist von der Formgedächtnistemperatur, auf die das Formgedächtniswerkstück ursprünglich erwärmt wurde. Nach einer vorbestimmten Haltedauer des Formgedächtniswerkstücks bei der von der Formgedächtnistemperatur verschiedenen Zwischentemperatur, wird das Formgedächtniswerkstück wieder auf die Formgedächtnistemperatur gebracht, also jeweils durch Erwärmen oder Abkühlen, wobei dabei im Wesentlichen und bevorzugterweise genau wieder die Formgedächtnistemperatur erreicht wird, welche der Formgedächtnistemperatur entspricht, auf die das Formgedächtniswerkstück ursprünglich gebracht wurde. Anschließend kann das Formgedächtniswerkstück ein weiteres Mal aufgeweitet werden.

Vorteilhafterweise ermöglicht das erfindungsgemäße Verfahren zur Formgebung eines Formgedächtniswerkstücks, indem mehrere Schritte der Aufweitung des Formgedächtniswerkstücks durchgeführt werden, dass das Aufweiten des Formgedächtniswerkstücks ausgehend von einem initialen bzw. ersten Durchmesser hin zu einem Zieldurchmesser, hier ein dritter Durchmesser, besonders schonend für das Formgedächtniswerkstück abläuft. Folglich kann selbst bei einem großen Durchmesserverhältnis zwischen dem Zieldurchmesser, der vor dem finalen Abkühlen erreicht wird, und dem initialen bzw. ersten Durchmesser, Schädigungen am Formgedächtniswerkstück vermindert bzw. verhindert können. Zugleich wird durch das Anwenden mehrerer Schritte zum Aufweiten, die Gefahr des Rückfederns der Geometrie des Formgedächtniswerkstücks, welches besonders bei einzelnen Schritten des Aufweitens mit großem Durchmesserverhältnis auftritt, vorteilhaft verhindert. Dies ermöglicht in der Folge, dass temperaturbedingte Formgedächtniseigenschaften bzw. superelastische Eigenschaften dem Formgedächtniswerkstück auf sehr genaue Weise eingeprägt werden können, insbesondere hinsichtlich einer Geometrie bzw. eines Durchmessers, den das Formgedächtniswerkstück bei einer bestimmten Umgebungstemperatur, im Sinne des Formgedächtnis, konfiguriert ist einzunehmen, indem es auf die sogenannte Austenit-finish-Temperatur erwärmt wird, oberhalb der das Gefüge des Formgedächtniswerkstücks im spannungslosen Zustand vollständig austenitisch vorliegt. Dadurch eignet sich das vorliegende Verfahren insbesondere als Verfahren zur Formgebung von Formgedächtniswerkstücken, die hohen Anforderungen an die Genauigkeit ihrer Verformung, ihrer Geometrie, sowie ihrer Werkstoff- und Produkteigenschaften innerhalb des Formgedächtnisses unterliegen, wie beispielhaft im medizinischen Bereich, beispielsweise für Stents, Herzklappen, Herzklappenrahmen oder ähnliche Teile. Die Eignung des vorliegenden Verfahrens ist jedoch nicht darauf beschränkt.

Weiterhin fördert das explizite Ändern der Temperatur des Formgedächtniswerkstücks auf die Zwischentemperatur, welche in bevorzugten Ausführungsformen unterhalb von 500 °C liegt, bevorzugt im Bereich von etwa 250 °C bis etwa 500 °C liegt, oder oberhalb von 525 °C liegt, bevorzugt im Bereich von etwa 525 °C bis etwa 600 °C, das Bilden von Ausscheidungen im Gefüge des Formgedächtniswerkstücks, was wiederum die definierte Einprägung superelastischer Eigenschaften bzw. der Formgedächtniseigenschaften begünstigt. Das erste Ändern der Temperatur des Formgedächtniswerkstücks kann in beispielhaften Ausführungsformen das Abkühlen des Formgedächtniswerkstücks auf eine Zwischentemperatur unterhalb der Formgebungstemperatur umfassen. In anderen beispielhaften Ausführungsformen kann das erste Ändern der Temperatur des Formgedächtniswerkstücks das Weitererwärmen des Formgedächtniswerkstücks auf eine Zwischentemperatur oberhalb der Formgebungstemperatur umfassen. Folglich kann das Bringen des Formgedächtniswerkstücks auf die Formgebungstemperatur, für den Fall des Abkühlens auf die Zwischentemperatur, ein erneutes Erwärmen des Formgedächtniswerkstücks auf die Formgebungstemperatur umfassen, sowie für den Fall des Weitererwärmens auf die Zwischentemperatur, ein Abkühlen des Formgedächtniswerkstücks auf die Formgebungstemperatur umfassen.

Darüber hinaus ermöglicht das vorliegende Verfahren vorteilhaft, eine besonders effiziente Formgebung für ein Formgedächtniswerkstück bereitzustellen, insbesondere in energetischer und zeitlicher Hinsicht. Indem das Formgedächtniswerkstück von dem Formgebungswerkzeug abgeworfen wird, wie beispielsweise in eine Abkühleinrichtung, kann das finale Abkühlen im Wesentlichen auf das Formgedächtniswerkstück gerichtet werden, während das Formgebungswerkzeug im Vergleich zum Formgedächtniswerkstück nur geringfügig oder gar nicht abgekühlt wird. Da das Formgebungswerkzeug vorteilhaft nicht mit abgeworfen wird, muss bei einem weiteren Durchlauf zur Formgebung eines weiteren Formgedächtniswerkstücks das Formgebungswerkzeug weder neu montiert, noch gänzlich wieder mit erwärmt werden, da es bereits gegenüber einem neu bereitgestellten Formgedächtniswerkstück eine höhere Temperatur aufweist. Dies ermöglicht somit vorteilhaft ein besonders zeit-, energie- und damit auch kosteneffizientes Verfahren zur Formgebung eines Formgedächtniswerkstücks.

Ebenso ermöglicht das vorliegende Verfahren, indem das Formgedächtniswerkstück von dem Formgebungswerkzeug abgeworfen wird, dass das Formgedächtniswerkstück einer besonders genau definierten Abkühlrate unterworfen werden kann, da dem Formgedächtniswerkstück nicht die thermisch träge Masse des Formgebungswerkzeugs anhaftet.

Somit ermöglicht das vorliegende Verfahren ein Formgedächtniswerkstück auf besonders effiziente Weise derart zu Formen, dass Formgedächtniseigenschaften bzgl. Durchmesser, Temperatur, sowie Material- und Produkteigenschaften des Formgedächtniswerkstücks gezielt eingestellt werden können, wodurch ein verbessertes Verfahren zur Formgebung eines Formgedächtniswerkstücks bereitgestellt wird.

In beispielhaften Ausführungsformen des Verfahrens zur Formgebung eines Formgedächtniswerkstücks kann der Schritt Abwerfen des Formgedächtniswerkstücks von dem Formgebungswerkzeug, insbesondere das Abwerfen des Formgedächtniswerkstücks von dem Formgebungswerkzeug in eine Abkühleinrichtung umfassen. Die Abkühleinrichtung kann beispielhaft ein Becken oder Bereich sein, das bzw. der mit einem Kühlmedium (insbesondere einer Kühlflüssigkeit) versehen ist. Beispielsweise kann die Abkühleinrichtung ein Wasserbecken umfassen bzw. sein.

In weiteren beispielhaften Ausführungsformen des vorliegenden Verfahrens kann das Verfahren nach dem Schritt des zweiten Aufweitens, einen Schritt eines zweiten Änderns der Temperatur des Formgedächtniswerkstücks auf eine Zwischentemperatur unterhalb oder oberhalb der Formgebungstemperatur aufweisen, und beispielhaft im Anschluss daran einen weiteren Schritt des erneuten Bringens des Formgedächtniswerkstücks auf die Formgebungstemperatur aufweisen, und darüber hinaus beispielhaft wiederum im Anschluss einen Schritt drittes Aufweiten des Formgedächtniswerkstücks auf einen vierten Durchmesser, der größer ist als der dritte Durchmesser, aufweisen.

Dadurch wird ermöglicht gegenüber dem Verfahren, bei dem im Anschluss an das zweite Aufweiten das Formgedächtniswerkstück abgeworfen wird und final abgekühlt wird, das Formgedächtniswerkstück weiter aufzuweiten und einen noch größeren Durchmesser für das Formgedächtniswerkstück einzuprägen, während insbesondere die Gefahr einer Beschädigung des Formgedächtniswerkstücks bei der Verformung bzw. beim Aufweiten verringert wird.

In weiteren beispielhaften Ausführungsformen des Verfahrens kann das Verfahren eine beliebige Anzahl weiterer Schritte des Änderns der Temperatur des Formgedächtniswerkstücks auf eine Zwischentemperatur unterhalb oder oberhalb der Formgebungstemperatur, beliebige (d.h. eine beliebige Anzahl von) weitere Schritte des Bringens des Formgedächtniswerkstücks auf die Formgebungstemperatur, und/oder beliebige (d.h. eine beliebige Anzahl von) weitere Schritte des Aufweitens auf einen Durchmesser, der gegenüber dem vorhergehenden Schritt des Aufweitens größer ist, aufweisen, bis im Wesentlichen ein gewünschter Zieldurchmesser erreicht wird bzw. ist. Dieser Zieldurchmesser stellt dabei bevorzugt etwa den Durchmesser dar, den das Formgedächtniswerkstück im Zuge einer Formgedächtnisverformung erreichen soll. Beispielsweise also den Durchmesser, den ein Stent konfiguriert ist einzunehmen, wenn er etwa auf Körpertemperatur erwärmt wird. Bevorzugt stellt der Zieldurchmesser einen Durchmesser dar, welcher zur Kompensation eines Rückfederns des Formgedächtniswerkstücks beim finalen Abkühlen, gegenüber dem Durchmesser, den das Formgedächtniswerkstück beim beispielhaften Einsetzen in einen Körper konfiguriert ist einzunehmen, leicht vergrößert ist. Da die konkreten Anwendungen von Formgedächtniswerkstücken verschieden ausfallen, beispielsweise hinsichtlich des genauen Durchmessers eines Gefäßes (z.B. einer Ader) zur Behandlung mit einem Stent, kann mittels einer wiederholbaren Schrittabfolge wie oben beschrieben, ein beliebiger gewünschter Durchmesser bzw. eine beliebige gewünschte Form, die das Formgedächtniswerkstück bei einer bestimmten Temperatur konfiguriert ist einzunehmen, als Formgedächtnis bzw. als superelastische Eigenschaft dem Formgedächtniswerkstück gezielt eingeprägt werden. Weiterhin ermöglicht die oben beschriebene wiederholbare Schrittabfolge, definierte Produkteigenschaften, wie beispielsweise die vom Formgedächtniswerkstück ausgeübte Kraft, insbesondere die Kraft in radialer Richtung beim Beispiel für Stents, bei einer bestimmten Deformation, sowie den Anteil einer plastischen Dehnung bei einer bestimmten Deformation, dem Formgedächtniswerkstück einzuprägen.

Zusätzlich ermöglicht das Abwerfen des Formgedächtniswerkstücks nach dem Aufweiten auf einen Zieldurchmesser insbesondere, dass im Wesentlichen nur das Formgedächtniswerkstück selbst abgekühlt wird, so dass das Formgedächtniswerkstück einer besonders genau definierten Abkühlrate unterworfen werden kann, und darüber hinaus ein vorteilhaft effizientes (insbesondere zeit- und kosteneffizientes) Verfahren bereitgestellt wird. Eine definierte Abkühlrate ermöglicht im Weiteren das Einprägen genau definierter Formgedächtniseigenschaften bzw. superelastischer Eigenschaften auf das Formgedächtniswerkstück.

In beispielhaften Ausführungsformen des Verfahrens zur Formgebung eines Formgedächtniswerkstücks kann das Aufweiten insbesondere ein radiales Aufweiten des Formgedächtniswerkstücks bzw. ein radiales Vergrößern eines Durchmessers des Formgedächtniswerkstücks umfassen, bevorzugt ein radial im Wesentlichen gleichmäßiges Aufweiten bzw. Vergrößern eines Durchmessers des Formgedächtniswerkstücks. Weiterhin kann das Aufweiten beispielhaft ein Aufweiten mittels des Formgebungswerkzeugs umfassen.

In weiteren beispielhaften Ausführungsformen kann das finale Abkühlen des Formgedächtniswerkstücks ein Abschrecken des Formgedächtniswerkstücks umfassen. Bevorzugt kann das Formgedächtniswerkstück beim finalen Abkühlen einen Durchmesser einnehmen, der im Wesentlichen dem Durchmesser entspricht, den das Formgedächtniswerkstück einnimmt, wenn das Formgedächtniswerkstück, beispielsweise nach Einbringung mittels eines Katheters in den menschlichen Körper und durch Aufwärmen über die Austenit-finish-Temperatur, einnimmt.

Weiterhin kann bzw. können in beispielhaften Ausführungsformen der Schritt bzw. die Schritte des Erwärmens des Formgedächtniswerkstücks auf die Formgebungstemperatur, das Erwärmen des Formgedächtniswerkstücks sowie das Erwärmen des Formgebungswerkzeugs jeweils auf die Formgebungstemperatur umfassen. So können beispielsweise sowohl Formgedächtniswerkstück als auch Formgebungswerkzeug, beispielhaft in einem Salzbad, gemeinsam erwärmt werden. Alternativ oder zusätzlich kann eines von dem Formgedächtniswerkstück und dem Formgebungswerkzeug derart erwärmt werden, dass sich das andere von dem Formgedächtniswerkstück und dem Formgebungswerkzeug mittelbar bzw. indirekt erwärmt.

Im weiteren Verlauf werden verschiedene Begriffe wiederholt verwendet, deren Verständnis durch die nachfolgenden beispielhaften Umschreibungen erleichtert werden soll.

Formgedächtniswerkstück: Der Begriff Formgedächtniswerkstück bezieht sich insbesondere auf ein Teil bzw. Bauteil, welches ein Formgedächtnismaterial aufweist bzw. daraus besteht, so dass das Formgedächtniswerkstück zumindest abschnittsweise Formgedächtniseigenschaften und/oder superelastische Eigenschaften aufweist. Beispielhafte Formgedächtniswerkstücke können dabei insbesondere Stents, Herzklappen und andere Teile sein. Die für die hier beschriebenen Aspekte und Ausführungsformen beschriebenen Formgedächtniswerkstücke können beliebige Formgedächtnismaterialien aufweisen bzw. daraus bestehen.

Superelastische Eigenschaften beschreiben eine durch äußere Krafteinwirkung verursachte reversible Formänderung. Eine superelastische Verformung aufgrund superelastischer Eigenschaften kann die Elastizität gewöhnlicher Metalle um ein Vielfaches übersteigen. Die Ursache dieses Verhaltens ist eine Phasenumwandlung innerhalb des Formgedächtnismaterials. Ausgehend von einem austenitischen Gefüge, wandelt sich das Gefüge unter Spannung zu einem martensitischen Gefüge um. Durch die Umwandlung kann das Formgedächtniswerkstück bei einer vorbestimmten Spannung mit verschiedenen Dehnungen vorliegen, wobei bei gleicher Spannung die Dehnung des austenitischen Gefüges geringer ist als die des martensitischen Gefüges. Bei Entlastung wandelt sich der Martensit wieder in Austenit um. Dafür sind keine Temperaturänderungen erforderlich. Demgegenüber werden temperaturbedingte Formgedächtniseigenschaften unterschieden. Ausgehend von einem Formgedächtnismaterial, welches als martensitischen Gefüge vorliegt, kann durch Erwärmen eine Phasenumwandlung in ein austenitisches Gefüge vollzogen werden. Diese Umwandlung ist reversibel. Ab einer sogenannten Austenit-finish-Temperatur liegt das Gefüge vollständig als austenitisches Gefüge vor. Diese Austenit-finish-Temperatur wird am Beispiel für Stents und Herzklappen vorzugsweise beim Einführen dessen in den menschlichen Körper erreicht und überschritten. Durch thermisch geführte Formgebungsverfahren können gewünschte superelastische Eigenschaften und Formgedächtniseigenschaften aufgeprägt werden.

Beispielhafte Formgedächtnismaterialien können Nickel-Titan Legierungen, insbesondere Nitinol, sein. Alternativ kann das Formgedächtnismaterial eine ternäre Nickel-Titan Legierung NiTiX sein, wobei X beispielsweise für Kupfer, Eisen, Niob oder Chrom steht, oder ein Polymer, oder eine beliebige andere Formgedächtnislegierung sein.

Formgebungstemperatur: Die Formgebungstemperatur beschreibt insbesondere einen Temperaturbereich, in dem ein Formgedächtniswerkstück, wenn es für eine vorbestimmte Zeit in einer bestimmten aufgezwungenen Form bzw. Geometrie einer Temperatur dieses Bereichs ausgesetzt ist, diese aufgezwungene Form bzw. Geometrie beibehält, selbst wenn die Zwangsbedingung nach der vorbestimmten Zeit gelöst wird. Die für das vorliegende Verfahren beispielhafte Formgebungstemperatur, wenn das Formgedächtniswerkstück Nitinol aufweist bzw. daraus besteht, liegt in bevorzugten Ausführungsformen in einem Bereich von etwa 425 °C bis etwa 550 °C. Die Formgebungstemperatur, auf die das Formgedächtniswerkstück gebracht wird, beeinflusst dabei Größe und Zusammensetzung der Ausscheidungen die sich im Gefüge des Formgedächtniswerkstücks bilden können, welche insbesondere NiTi₂ und/oder Ni₄Ti₃ umfassen können.

In beispielhaften Ausführungsformen kann der untere Formgebungstemperaturbereich verfahrenstechnisch mit einem Erwärmen auf eine Zwischentemperatur, welche oberhalb der Formgebungstemperatur liegt, verknüpft werden. In alternativen Ausführungsformen kann der untere Formgebungstemperaturbereich verfahrenstechnisch auch mit einem Abkühlen auf eine Zwischentemperatur, welche unterhalb der Formgebungstemperatur liegt, verknüpft werden.

Weiterhin kann in beispielhaften Ausführungsformen der obere Formgebungstemperaturbereich verfahrenstechnisch mit einem Abkühlen auf eine Zwischentemperatur, welche unterhalb der Formgebungstemperatur liegt, verknüpft werden. In alternativen Ausführungsformen kann der obere Formgebungstemperaturbereich verfahrenstechnisch auch mit einem Weitererwärmen auf eine Zwischentemperatur, welche oberhalb der Formgebungstemperatur liegt, verknüpft werden.

Axiale Richtung: Die axiale Richtung wird vorliegend insbesondere hauptsächlich zur Beschreibung des Formgebungswerkzeugs verwendet, welches sich mit einem Teil seiner Komponenten im Wesentlichen in einer axialen Richtung erstreckt. Die axiale Richtung kann dabei insbesondere eine Richtung sein, welche einer Wesentlichen Erstreckung eines Verfahrrohrs und/oder eines Führungselements des Formgebungswerkzeugs entspricht. Die axiale Richtung kann weiterhin eine Richtung sein, entlang der das Verfahrrohr verfahrbar ist, und/oder eine Richtung sein, entlang der benachbarte Scheiben des Formgebungswerkzeugs zueinander beabstandet sind. Darüber hinaus kann die axiale Richtung insbesondere eine Richtung sein, welche einer wesentlichen Erstreckung und/oder im Wesentlichen einer Achse eines zylinderförmigen bzw. annäherungsweise zylinderförmigen Formgedächtniswerkstücks entsprechen.

Radiale Richtung: Die radiale Richtung wird vorliegend insbesondere hauptsächlich im Zusammenhang mit dem Durchmesser des Formgedächtniswerkstücks verwendet, und beschreibt die wesentliche Verformung des Formgedächtniswerkstücks bei dem einen oder der mehreren Schritte des Aufweitens, beispielsweise durch ein Formgebungswerkzeug. Die radiale Richtung kann bevorzugt im Wesentlichen senkrecht zur axialen Richtung verlaufen. Mit anderen Worten kann die radiale Richtung insbesondere im Wesentlichen einer radialen Richtung eines zylinderförmigen bzw. annäherungsweise zylinderförmigen Formgedächtniswerkstücks entsprechen.

Eine Umfangsrichtung kann bevorzugt im Wesentlichen senkrecht zur axialen Richtung und/oder im Wesentlichen senkrecht zur radialen Richtung verlaufen.

Die axiale Richtung, die radiale Richtung und die Umfangsrichtung können ein Koordinatensystem bilden, insbesondere ein Rechtssystem.

Wird eine Richtung oder ein Winkel mit dem Zusatz "im Wesentlichen" oder "in etwa" wiedergegeben, so sei mit diesem Zusatz insbesondere eine Abweichung von der betreffenden Richtung bzw. vom betreffenden Winkel im Bereich von 0° bis 5° gemeint bzw. zu verstehen.

Wird ein räumliches Maß, ein räumliches Verhältnis oder ein sonstiges Verhältnis mit dem Zusatz "im Wesentlichen" oder "in etwa" wiedergegeben, so sei mit diesem Zusatz insbesondere eine Abweichung von dem betreffenden Maß bzw. dem betreffenden Verhältnis im Bereich von 0 % bis 10 % gemeint bzw. zu verstehen.

Wird eine Temperatur mit dem Zusatz "im Wesentlichen" oder "in etwa" wiedergegeben, so sei mit diesem Zusatz insbesondere eine Abweichung von der betreffenden Temperatur im Bereich von 0 % bis 5 % bzgl. seines Äquivalents in Kelvin gemeint bzw. zu verstehen. Unter dem Begriff "Raumtemperatur" bzw. "etwa Raumtemperatur" sei insbesondere eine Temperatur im Bereich von etwa 15 °C bis etwa 25 °C zu verstehen. Unter dem Begriff "etwa Körpertemperatur" sei eine Temperatur im Bereich von etwa 33 °C bis etwa 42 °C zu verstehen.

In bevorzugten Ausführungsformen des Verfahrens zur Formgebung eines Formgedächtniswerkstücks, kann das erste Ändern der Temperatur des Formgedächtniswerkstücks auf eine Zwischentemperatur ein erstes Abkühlen des Formgedächtniswerkstücks auf die Zwischentemperatur unterhalb der Formgebungstemperatur umfassen, oder ein erstes Weitererwärmen des Formgedächtniswerkstücks auf die Zwischentemperatur oberhalb der Formgebungstemperatur umfassen, und das erste Ändern der Temperatur des Formgedächtniswerkstücks bevorzugt das Ändern der Temperatur des Formgedächtniswerkstücks um zumindest etwa 25 °C, vorzugsweise um zumindest etwa 40 °C, weiter vorzugsweise um zumindest etwa 50 °C umfassen.

Vorteilhafterweise fördert das Ändern der Temperatur des Formgedächtniswerkstücks auf die Zwischentemperatur, durch ein Abkühlen oder ein Weitererwärmen des Formgedächtniswerkstücks, das Bilden von Ausscheidungen im Gefüge des Formgedächtniswerkstücks, was wiederum die definierte Einprägung superelastischer Eigenschaften bzw. der Formgedächtniseigenschaften begünstigt. Das Ändern der Temperatur des Formgedächtniswerkstücks um zumindest etwa 25 °C, bevorzugt um zumindest etwa 40 °C, weiter bevorzugt um zumindest etwa 50 °C verstärkt den vorgenannten Effekt des Bildens von Ausscheidungen weiterhin.

So kann gemäß einer beispielhaften Ausführungsform das Formgedächtniswerkstück beim Schritt des Erwärmens des Formgedächtniswerkstücks auf die Formgebungstemperatur beispielsweise auf eine Temperatur von etwa 500 °C erwärmt werden, und im Schritt des ersten Änderns der Temperatur des Formgedächtniswerkstücks auf eine Zwischentemperatur unterhalb oder oberhalb der Formgebungstemperatur beispielsweise auf eine Temperatur von etwa 465 °C abgekühlt werden.

In bevorzugten Ausführungsformen des Verfahrens zur Formgebung eines Formgedächtniswerkstücks kann der zweite Durchmesser das etwa 1,5-fache bis etwa 1,9-fache des ersten Durchmessers sein, bevorzugt das etwa 1,85-fache des ersten Durchmessers sein. Zusätzlich oder alternativ kann der dritte Durchmesser das etwa 1,5-fache bis etwa 1,9-fache des zweiten Durchmessers sein, bevorzugt das etwa 1,85-fache des zweiten Durchmessers sein.

Vorteilhaft ermöglichen die vorgenannten Durchmesserverhältnisse, welche insbesondere bei den Schritten des Aufweitens erreicht werden, dass ausgehend von einem initialen bzw. ersten Durchmesser eines Formgedächtniswerkstücks und zur Erreichung eines großen Zieldurchmessers des Formgedächtniswerkstücks, einerseits nur wenige Schritte bzw. eine sehr begrenzte Anzahl an Schritten des Aufweitens benötigt werden, und andererseits Schädigungen am Formgedächtniswerkstück verringert bzw. vermieden werden können. Folglich ermöglichen die vorgenannten Durchmesserverhältnisse, dass die Formgedächtniseigenschaften aus Durchmesser bzw. Form und Temperatur des Formgedächtniswerkstücks zielgenau dem Formgedächtniswerkstück eingeprägt werden können. Die vorgenannten Durchmesserverhältnisse sind insbesondere auch für Schritte des Aufweitens geeignet, welche über das erste Aufweiten und das zweite Aufweiten hinausgehen, also beispielsweise für ein drittes Aufweiten, viertes Aufweiten usw. Mit anderen Worten ermöglichen die vorstehend genannten bevorzugten Durchmesserverhältnisse einen vorteilhaften Kompromiss aus Prozesssicherheit zur Definition der gewünschten Produkteigenschaften des Formgedächtniswerkstücks, und Prozessökonomie, indem nur wenige einzelne Schritte zum Aufweiten des Formgedächtniswerkstücks benötigt werden.

In beispielhaften Ausführungsformen können die vorgenannten Durchmesserverhältnisse auch über- oder unterschritten werden, um einen möglichst genauen Zieldurchmesser für das Formgedächtniswerkstück einzustellen. Wird ein Durchmesserverhältnis von 1,9 unterschritten, können beim Aufweiten des Formgedächtniswerkstücks dabei vorteilhaft kritische lokal überhöhte Dehnungen am Formgedächtniswerkstück verringert bzw. vermieden werden, und dadurch weiter Schädigungen am Formgedächtniswerkstück eingeschränkt bzw. vermieden werden.

In bevorzugten Ausführungsformen des Verfahrens zur Formgebung eines Formgedächtniswerkstücks kann das erste Aufweiten und das zweite Aufweiten ein Aufweiten des Formgedächtniswerkstücks in radialer Richtung, insbesondere unter Beibehaltung der axialen Erstreckung des Formgedächtniswerkstücks, umfassen.

Vorteilhaft ermöglicht die Beibehaltung der axialen Erstreckung des Formgedächtniswerkstücks, während des Aufweitens, dass sich die Formänderung am Formgedächtniswerkstück durch das Aufweiten in radialer Richtung im Wesentlichen bzw. nur auf den Durchmesser des Formgedächtniswerkstücks bezieht. Mit anderen Worten wird vorteilhaft ermöglicht, dass während dem Aufweiten keine oder nur eine geringfügige Stauchung des Formgedächtniswerkstücks auftritt, welche die gewünschte Geometrie hinsichtlich des Formgedächtnisses negativ beeinträchtigt. Die Beibehaltung der wesentlichen axialen Erstreckung des Formgedächtniswerkstücks beim Aufweiten ermöglicht darüber hinaus, dass aufwendige und komplizierte Nacharbeiten am Formgedächtniswerkstück vermieden werden können.

Unter der Beibehaltung der axialen Erstreckung des Formgedächtniswerkstücks, ist insbesondere die Vermeidung einer unkontrollierten axialen Deformation des Formgedächtniswerkstücks, während dem Aufweiten in radialer Richtung, zu verstehen. Bevorzugt wird im vorliegenden Verfahren die axiale Deformation derart begrenzt, dass das Formgedächtniswerkstück nach dem Aufweiten zumindest das etwa 0,70-fache, bevorzugt das etwa 0,80-fache, weiter bevorzugt das etwa 0,90-fache, noch weiter bevorzugt zumindest das etwa 0,95-fache, und besonders bevorzugt zumindest das etwa 0,98-fache seiner axialen Erstreckung vor dem Aufweiten aufweist. In Abhängigkeit des konkreten Formgedächtniswerkstück und des aufgebrachten Umformgrades am konkreten Formgedächtniswerkstück können jedoch auch davon abweichende Grenzen axialer Deformation zugelassen werden bzw. sein.

In beispielhaften Ausführungsformen kann jeder Schritt des Aufweitens ein Aufweiten des Formgedächtniswerkstücks, unter Beibehaltung der axialen Erstreckung des Formgedächtniswerkstücks, umfassen.

In weiteren beispielhaften Ausführungsformen kann das Aufweiten des Formgedächtniswerkstücks insbesondere auch ein Aufweiten des Formgedächtniswerkstücks in radialer Richtung, unter Beibehaltung der axialen Anordnung des Formgedächtniswerkstücks an dem Formgebungswerkzeug, umfassen.

Insbesondere ermöglicht dies weiter vorteilhaft, dass während dem Aufweiten nicht nur die axiale Erstreckung bzw. die axiale Geometrie des Formgedächtniswerkstücks definiert eingestellt werden kann, sondern auch die Anordnung des Formgedächtniswerkstücks an dem Formgebungswerkzeug erhalten bleibt. Dadurch wird weiter vorteilhaft ermöglicht, dass das Formgedächtniswerkstück an einem vorbestimmten Bereich des Formgebungswerkzeugs angeordnet werden kann, von dem es durch das Aufweiten nicht verschoben wird. Damit können die Schritte des Abkühlens und/oder des Erwärmens in einem gezielt eingeschränkten Bereich vorgenommen werden.

Ferner ermöglicht dies insbesondere ein energetisch besonders effizientes Verfahren zur Formgebung eines oder mehrerer Formgedächtniswerkstücke bereitzustellen, wobei gerade die thermisch relevanten und damit energetisch intensiven Schritte des Abkühlens und/oder des Erwärmens auf den Bereich konzentriert werden können, in dem das Formgedächtniswerkstück an dem Formgebungswerkzeug angeordnet ist, unabhängig der genauen Anzahl der vorgenommenen Schritte bzw. Zyklen aus Erwärmen, Aufweiten und/oder Abkühlen.

In beispielhaften Ausführungsformen kann das Beibehalten der axialen Erstreckung des Formgedächtniswerkstücks und/oder das Beibehalten der axialen Anordnung des Formgedächtniswerkstücks an dem Formgebungswerkzeug durch ein formschlüssiges und/oder reibschlüssiges Halten sichergestellt werden, wie beispielsweise durch spannende bzw. haltende Haken, Anschläge, Greifer oder ähnliches.

In bevorzugten Ausführungsformen des Verfahrens zur Formgebung eines Formgedächtniswerkstücks kann während zumindest einem des ersten und des zweiten Aufweitens das Formgedächtniswerkstück reibschlüssig gehalten sein, insbesondere reibschlüssig an dem Formgebungswerkzeug gehalten sein.

Darüber hinaus kann in beispielhaften Ausführungsformen in bzw. während jedem Schritt des Aufweitens das Formgedächtniswerkstück reibschlüssig gehalten bzw. lokalisiert sein.

Vorteilhaft ermöglicht das reibschlüssige Halten bzw. Lokalisieren des Formgedächtniswerkstücks, während dem Aufweiten, dass das Formgedächtniswerkstück an dem Formgebungswerkzeug auf besonders einfache Weise gehalten sein kann.

In weiteren beispielhaften Ausführungsformen kann das reibschlüssige Halten bzw. Lokalisieren insbesondere ein reibschlüssiges Halten bzw. Lokalisieren unter Beibehaltung der axialen Erstreckung des Formgedächtniswerkstücks, besonders bevorzugt unter Beibehaltung der axialen Position des Formgedächtniswerkstücks an dem Formgebungswerkzeug, umfassen.

Das reibschlüssige Halten bzw. Lokalisieren des Formgedächtniswerkstücks ermöglicht das Halten des Formgedächtniswerkstücks in seiner axialen Erstreckung und/oder in seiner axialen Anordnung an dem Formgebungswerkzeug auf besonders einfache Weise, beispielsweise unter Verwendung einer vorbestimmten Materialpaarung, sicherzustellen.

In bevorzugten Ausführungsformen kann das reibschlüssige Halten bzw. Lokalisieren auf einer Haftreibung basieren, insbesondere auf einer Haftreibung zwischen dem Formgedächtniswerkstück und dem Formgebungswerkzeug.

Insbesondere ermöglicht die Haftreibung zwischen Formgedächtniswerkstück und Formgebungswerkzeug vorteilhaft, dass das Aufweiten des Formgedächtniswerkstücks mit einem möglichst großen Aufweitungswinkel, beispielsweise im Vergleich zu einer Gleitreibung, durchgeführt werden kann. Dies ermöglicht weiter vorteilhaft, dass das Verfahren zur Formgebung des Formgedächtniswerkstücks zusätzlich besonders zeiteffizient durchgeführt werden kann, sowie im Weiteren eine kompakte Struktur für das Formgebungswerkzeug, was wiederum eine erhöhte Energieeffizienz bewirkt.

In besonders bevorzugten Ausführungsformen kann der Reibschluss zwischen einem Formgedächtniswerkstück das Nitinol aufweist bzw. daraus besteht und einer Vielzahl von Aufweitungsdrähten bzw. Aufweitungselementen die Nitinol aufweisen bzw. daraus bestehen konfiguriert sein. Die Materialpaarung Nitinol-Nitinol zur Konfiguration eines reibschlüssigen Haltens des Formgedächtniswerkstücks verhindert vorteilhaft die Verunreinigung des zu formenden Formgedächtniswerkstücks auch bei hohen Temperaturen, verhindert weiter die Verunreinigung eines möglichen Salzbades zur Erwärmung des Formgedächtniswerkstücks, und ermöglicht darüber hinaus eine temperaturunempfindliche, hohe Haftreibung. Weiterhin stellt ein Formgebungswerkzeug, welches Nitinol aufweist, eine vorteilhafte Elastizität in einem großen Temperaturbereich bereit. Ein bevorzugter Aufweitungswinkel, gemessen gegenüber einer im Wesentlichen axialen Richtung, kann für eine Materialpaarung Nitinol-Nitinol insbesondere im Bereich von etwa 7° bis etwa 20° liegen, bevorzugt im Bereich von etwa 10° bis etwa 16° liegen.

In alternativen Ausführungsformen können davon abweichende Materialpaarungen zur Konfiguration eines Reibschlusses verwendet werden.

In bevorzugten Ausführungsformen des Verfahrens zur Formgebung eines Formgedächtniswerkstücks kann das Formgedächtniswerkstück:
- eine Nickel-Titan Legierung aufweisen; und/oder
- eine runde Form aufweisen; und/oder
- ein Stentmuster aufweisen.

Durch ein Formgedächtniswerkstück, welches eine Nickel-Titan Legierung, wie beispielsweise Nitinol aufweist bzw. daraus besteht, kann das Formgedächtniswerkstück insbesondere für medizinische Anwendungen geeignet sein.

Durch eine bevorzugt runde Form des Formgedächtniswerkstücks kann dieses besonders einfach gleichmäßig in radialer Richtung, also auf einen bestimmten diskreten Durchmesser, aufgeweitet werden. Die Form bzw. Grundform des Formgedächtniswerkstücks kann insbesondere im Wesentlichen zylindrisch bzw. im Wesentlichen ringförmig sein, ist dabei jedoch nicht auf eine exakt runde Form beschränkt, sondern kann beispielsweise auch vieleckig oder oval sein.

Durch ein bevorzugtes Stentmuster ist das Formgedächtniswerkstück insbesondere zur medizinischen Anwendung bzw. zur Verwendung als Stent bzw. Implantat geeignet. Das Stentmuster kann dabei als metallisches Geflecht mit einem vorbestimmten Muster ausgebildet sein. Weiterhin kann das Stentmuster insbesondere durch Stanzen aus einer Metallplatte, sowie durch Materialabtragen (z.B. mittels Laserschneiden) einer Metallplatte oder eines Metallzylinders bzw. -rohrs, oder ähnlich geformt sein.

In alternativen Ausführungsformen kann das Formgedächtniswerkstück von einer runden Form und/oder einem Stentmuster abweichen bzw. eine davon jeweils abweichende Form aufweisen. Beispielsweise kann das Formgedächtniswerkstück eine beliebige Mehrkantform, beispielsweise ähnlich eines Mehrkantrohrs, aufweisen. Darüber hinaus kann das Formgedächtniswerkstück abschnittsweise Bereiche mit einem verringerten oder vergrößerten Durchmesser, Einschnürungen, Ein- oder Ausbeulungen sowie konische Bereiche aufweisen.

Ein weiterer Aspekt der Erfindung betrifft ein Formgebungswerkzeug zur Formgebung eines Formgedächtniswerkstücks, aufweisend:
- ein Führungselement, und
- ein Verfahrrohr, welches verfahrbar an dem Führungselement angeordnet ist, wobei
   -- an dem Verfahrrohr in vorbestimmten axialen Abständen Scheiben angeordnet sind,
   -- zwischen den Scheiben Aufweitungsdrähte bzw. Aufweitungselemente gespannt sind, und wobei
   -- die zwischen den Scheiben gespannten Aufweitungsdrähte bzw. Aufweitungselemente Durchmesser bilden, um ein Formgedächtniswerkstück umfänglich daran anzuordnen.

Vorteilhaft ermöglicht das vorliegende Formgebungswerkzeug, dass ein Formgedächtniswerkstück sicher an seinem Innenumfang mittels einer Vielzahl von Aufweitungsdrähten bzw. -elementen gehalten werden kann. Gleichzeitig ermöglicht der drahtartige bzw. elementartige Aufbau des Formgebungswerkzeugs, um das Formgedächtniswerkstück daran anzuordnen und im Weiteren auch aufzuweiten, dass das Formgebungswerkzeug insbesondere eine besonders geringe Wärmekapazität aufweist, so dass mittels des vorliegenden Formgebungswerkzeugs eine besonders energieeffiziente Formgebung eines Formgedächtniswerkstücks ermöglicht wird.

Darüber hinaus ermöglicht das vorliegende Formgebungswerkzeug, durch die Bereitstellung einer Vielzahl von Aufweitungsdrähten bzw. -elementen, um ein Formgedächtniswerkstück daran anzuordnen, dass eine entsprechende Vielzahl an diskreten Haltestellen bzw. Halterungsstellen zwischen dem Formgedächtniswerkstück und dem Formgebungswerkzeug bereitgestellt wird. Dadurch wird ein besonders sicheres und definiertes Halten, während eines beispielhaften Aufweitens des Formgedächtniswerkstücks, ermöglicht.

In beispielhaften Ausführungsformen des Formgebungswerkzeugs kann sich das Führungselement im Wesentlichen in einer Längsrichtung, insbesondere im Wesentlichen in einer axialen Richtung, erstrecken. Beispielsweise kann das Führungselement als Führungsstab, Führungsrohr oder ähnliches ausgebildet sein. Weiterhin kann das Verfahrrohr sich beispielhaft im Wesentlichen in einer axialen Richtung erstrecken, oder im Wesentlichen zumindest abschnittsweise parallel zum Führungselement erstrecken.

In weiteren beispielhaften Ausführungsformen können die Aufweitungsdrähte bzw. -elemente derart an den Scheiben angeordnet sein bzw. sich zwischen diesen erstrecken, dass das Formgedächtniswerkstück, wenn es am Formgebungswerkzeug angeordnet ist, ausschließlich von den Aufweitungsdrähten bzw. -elementen kontaktiert wird. Das Formgebungswerkzeug kann eine beliebige Vielzahl an Aufweitungsdrähten bzw. -elementen aufweisen, beispielsweise 3, 4, 5, 6, 7, 8, 9, 10, 11, 12, 13, 14, 15, 16, 17, 18, 19, 20, oder eine beliebige größere Vielzahl von Aufweitungsdrähten bzw. -elementen aufweisen.

In beispielhaften Ausführungsformen können die Aufweitungsdrähte bzw. -elemente im Wesentlichen entlang eines äußeren Umfangs der Scheiben angeordnet sein bzw. zumindest abschnittsweise entlang eines äußeren Umfangs der Scheiben verlaufen. So können die Aufweitungsdrähte bzw. -elemente beispielhaft derart an den Scheiben angeordnet sein, dass die Aufweitungsdrähte bzw. -elemente zumindest abschnittsweise gegenüber den Scheiben in radialer Richtung nach außen vorstehen. Mit anderen Worten können die Aufweitungsdrähte bzw. -elemente beispielhaft in ihrer Längserstreckung derart an den Scheiben angeordnet sein, dass sie mit ihrem Drahtdurchmesser zumindest abschnittsweise radial gegenüber dem Durchmesser der Scheiben, an denen sie angeordnet sind bzw. zwischen denen sie gespannt sind, vorstehen.

Die Aufweitungsdrähte bzw. -elemente können bevorzugt im Wesentlichen eine Gitterstruktur bilden, insbesondere eine Gitterstruktur zueinander in einer Umfangsrichtung beabstandeter länglicher Gitterelemente.

In Abhängigkeit der Geometrie der Scheiben zwischen denen die Aufweitungsdrähte bzw. -elemente gespannt sind bzw. zwischen denen sich die Aufweitungsdrähte bzw. -elemente erstrecken, bilden die Aufweitungsdrähte bzw. -elemente beispielhaft eine abschnittsweise Gitterstruktur. Die Gitterstruktur kann abschnittsweise aus einem oder mehreren zylinderförmigen Abschnitten und einem oder mehreren kegelstumpfförmigen Abschnitten gebildet sein. Insbesondere können sich die einen oder mehreren zylinderförmigen Abschnitte mit dem einen oder mehreren kegelstumpfförmigen Abschnitte gegenseitig abwechseln.

In beispielhaften Ausführungsformen können die Aufweitungsdrähte bzw. -elemente insbesondere eine Gitterstruktur bilden, welche einen ersten im Wesentlichen zylinderförmigen Abschnitt mit einem ersten diskreten Durchmesser aufweist, einen sich daran anschließenden ersten im Wesentlichen kegelstumpfförmigen Abschnitt aufweisen, der einen Übergang von dem ersten diskreten Durchmesser zu einem zweiten diskreten Durchmesser bildet, und einen zweiten im Wesentlichen zylinderförmigen Abschnitt mit dem zweiten diskreten Durchmesser aufweisen, der sich an den ersten im Wesentlichen kegelstumpfförmigen Abschnitt anschließt und größer ist als der erste diskrete Durchmesser.

Weitere beispielhafte Ausführungsformen können eine Gitterstruktur bilden, welche einen oder mehrere weitere im Wesentlichen kegelstumpfförmige Abschnitte aufweist, die jeweils einen Übergang zwischen zwei Durchmessern bilden, sowie einen oder mehrere weitere im Wesentlichen zylinderförmige Abschnitte aufweisen, die einen diskreten Durchmesser bilden, welcher größer ist als ein vorhergehender diskreter Durchmesser. Insbesondere können weitere beispielhafte Ausführungsformen mittels der Aufweitungsdrähte bzw. -elemente eine Gitterstruktur bilden, welche einen ersten im Wesentlichen zylinderförmigen Abschnitt mit einem ersten diskreten Durchmesser aufweist, einen sich daran anschließenden ersten im Wesentlichen kegelstumpfförmigen Abschnitt aufweisen, der einen Übergang von dem ersten diskreten Durchmesser zu einem zweiten diskreten Durchmesser bildet, einen zweiten im Wesentlichen zylinderförmigen Abschnitt mit dem zweiten diskreten Durchmesser aufweisen, der sich an den ersten im Wesentlichen kegelstumpfförmigen Abschnitt anschließt und größer ist als der erste diskrete Durchmesser, einen sich daran anschließenden zweiten im Wesentlichen kegelstumpfförmigen Abschnitt aufweisen, der einen Übergang von dem zweiten diskreten Durchmesser zu einem dritten diskreten Durchmesser bildet, sowie einen dritten im Wesentlichen zylinderförmigen Abschnitt mit dem dritten diskreten Durchmesser aufweisen, der sich an den zweiten im Wesentlichen kegelstumpfförmigen Abschnitt anschließt und größer ist als der zweite diskrete Durchmesser.

Der Übergang zwischen zwei diskreten Durchmessern kann besonders bevorzugt durch einen vorbestimmten Aufweitungswinkel konfiguriert sein, um insbesondere einen Reibschluss, vorzugsweise einen Reibschluss durch Haftreibung eines Formgedächtniswerkstücks an der Vielzahl von Aufweitungsdrähten bzw. -elementen sicherzustellen.

Der erste Durchmesser, den der erste zylinderförmige Abschnitt mit dem ersten Durchmesser bildet, kann insbesondere konfiguriert sein, um das Formgedächtniswerkstück unter einer Vorspannung daran zu halten. Die Vorspannung kann dabei derart geringfügig sein, dass sie lediglich dazu dient, dass das Formgedächtniswerkstück nicht von selbst abrutscht. Mit anderen Worten kann das Formgedächtniswerkstück insbesondere unter Aufbringung einer elastischen Deformation an dem ersten zylinderförmigen Abschnitt mit dem ersten Durchmesser anordenbar sein, wobei eine elastische Rückstellkraft des Formgedächtniswerkstücks, das Formgedächtniswerkstück an dem ersten zylinderförmigen Abschnitt hält.

Weiterhin zusätzlich oder alternativ können die Aufweitungsdrähte bzw. -elemente insbesondere eine Gitterstruktur bilden, welche abschnittsweise einen im ovalen, oder im Wesentlichen vieleckigen Querschnitt, wie beispielsweise einen hexagonalen Querschnitt, aufweist.

Die vorangehend beschriebenen beispielhaften und bevorzugten Ausführungsformen ermöglichen somit vorteilhaft ein Formgebungswerkzeug bereitzustellen, welches das schrittweise Aufweiten eines Formgedächtniswerkstücks auf einfache Weise ermöglicht. Weiterhin kann mittels des vorstehend beschriebenen Formgebungswerkzeugs ein Verfahren zur Formgebung eines Formgedächtniswerkstücks besonders energieeffizient durchgeführt werden, da das Formgebungswerkzeug durch seine gitterförmige Struktur eine besonders geringe Wärmekapazität aufweist.

In bevorzugten Ausführungsformen des Formgebungswerkzeugs zur Formgebung eines Formgedächtniswerkstücks kann das Formgebungswerkzeug einen Aufnahmebereich zur Anordnung eines Formgedächtniswerkstücks mit einem ersten Durchmesser daran aufweisen, und
- das Verfahrrohr konfiguriert sein mittels eines Aktors relativ zum Aufnahmebereich bewegt zu werden, so dass das Formgedächtniswerkstück im Aufnahmebereich von den Scheiben passierbar ist.

In beispielhaften Ausführungsformen kann der Aufnahmebereich durch Aufweitungsdrähte bzw. -elemente gebildet sein, welche einen ersten Aufnahmedurchmesser bilden, um ein Formgedächtniswerkstück aufweisend einen ersten bzw. initialen Durchmesser daran anzuordnen.

Mit anderen Worten kann der Aufnahmebereich insbesondere als initialer Aufnahmebereich konfiguriert sein, der zunächst einen ersten Aufnahmedurchmesser, zur Anordnung eines Formgedächtniswerkstücks aufweisend einen initialen bzw. ersten Durchmesser daran, ausbildet bzw. aufweist, und durch das Passieren der Scheiben konfiguriert ist einen Durchmesser auszubilden bzw. aufzuweisen, der entsprechend der passierenden Scheiben vergrößert oder verringert ist.

Durch das Passieren der Scheiben können sich die Aufweitungsdrähte bzw. -elemente insbesondere in radialer Richtung, gemäß des Durchmessers bzw. der Geometrie der passierenden Scheiben bewegen, wodurch die Vielzahl an in Umfangsrichtung verteilt angeordneten Aufweitungsdrähten bzw. -elementen einen der passierenden Scheibe entsprechenden Durchmesser bilden. Da das Formgedächtniswerkstück umfänglich an den Aufweitungsdrähten bzw. -elementen angeordnet wird, kann somit das Halten des Formgedächtniswerkstücks unabhängig von dem Vorgang der Verformung bzw. der Aufweitung des Formgedächtniswerkstücks einstellbar bzw. konfigurierbar sein.

Weiterhin vorteilhaft kann somit zur Verformung bzw. Aufweitung des Formgedächtniswerkstücks eine Relativbewegung zwischen Formgedächtniswerkstück und den Aufweitungsdrähten bzw. -elementen verhindert werden. Dadurch kann beispielhaft auf bevorzugte Weise ein Halten des Formgedächtniswerkstücks auf Basis einer Haftreibung zwischen dem Formgedächtniswerkstück und den Aufweitungsdrähten bzw. -elementen konfiguriert werden, das von den passierenden Scheiben, welche mittels des Aktors bewegt werden, unabhängig bzw. unbeeinflusst ist.

In bevorzugten Ausführungsformen des Formgebungswerkzeugs zur Formgebung eines Formgedächtniswerkstücks können die Scheiben derart geformt sein, dass das Formgebungswerkzeug konfiguriert ist mittels einer Bewegung des Verfahrrohrs entlang des Führungselements das Formgedächtniswerkstück aufzuweiten.

In beispielhaften Ausführungsformen kann das Verfahrrohr parallel zum Führungselement bzw. parallel zur Erstreckung des Führungselements verfahrbar sein, insbesondere entlang einer axialen Richtung verfahrbar sein.

In beispielhaften Ausführungsformen können die Scheiben derart geformt sein, dass beim Passieren des Aufnahmebereichs in axialer Richtung, die Scheiben die Aufweitungsdrähte bzw. -elemente radial aufweiten bzw. radial nach außen drücken. Mit anderen Worten könnten die an dem Verfahrrohr angeordneten Scheiben derart geformt sein, dass jede entgegen der axialen Richtung aufeinander folgende Scheibe jeweils mindestens den gleichen Durchmesser aufweist, wie die davor angeordnete Scheibe.

In beispielhaften Ausführungsformen können entgegen der axialen Richtung aufeinanderfolgend zunächst zumindest zwei Scheiben eines ersten Scheibendurchmessers angeordnet sein, und daran anschließend zumindest zwei Scheiben eines zweiten Scheibendurchmessers angeordnet sein, der größer ist als der erste Scheibendurchmesser. In weiteren beispielhaften Ausführungsformen können eine oder mehrere Scheiben zwischen den Scheiben ersten und zweiten Scheibendurchmessers angeordnet sein und einen Scheibendurchmesser aufweisen, der zwischen dem ersten und zweiten Scheibendurchmesser liegt. Die Anordnung weiterer Scheiben in diesem Sinne ist beispielhaft möglich.

Durch das Passieren der Scheiben mit vorbestimmtem Durchmesser, und bevorzugt mit einer vorbestimmten Geschwindigkeit, wird vorteilhaft ermöglicht, eine definierte Umformgeschwindigkeit, und damit vor allem eine definierte Kraft, bei der Formgebung bzw. beim Aufweiten auf das Formgedächtniswerkstück aufzubringen.

In bevorzugten Ausführungsformen des Formgebungswerkzeugs zur Formgebung eines Formgedächtniswerkstücks können die Scheiben entlang ihres Umfangs Gleitnuten aufweisen,
wobei entlang bzw. in jeder Gleitnut ein Aufweitungsdraht bzw. -element durch die jeweilige Scheibe gleitend geführt ist, und
wobei bevorzugt jeder Aufweitungsdraht bzw. -element derart in der jeweiligen Gleitnut angeordnet ist, dass er bzw. es radial nach außen gegenüber der Scheibe, in deren Gleitnut der Aufweitungsdraht bzw. -element geführt ist, vorsteht.

Vorteilhaft ermöglicht die gleitende Führung der Aufweitungsdrähte in Gleitnuten der Scheiben, dass lediglich die Scheiben bewegt werden müssen, um ein Aufweiten des Formgedächtniswerkstücks zu ermöglichen, und gleichzeitig eine Relativbewegung zwischen den Aufweitungsdrähten und dem Formgedächtniswerkstück vermieden wird, was wiederum ein Aufweiten mit einem großen Aufweitungswinkel ermöglicht, und im Weiteren eine axiale Verkürzung des Formgebungswerkzeugs ermöglicht, was wiederum die Energieeffizienz erhöht.

Die Gleitnuten können beispielsweise als Bohrungen, insbesondere als Bohrungen in axialer Richtung durch die Scheibe(n) hindurch, konfiguriert sein.

In beispielhaften Ausführungsformen weisen die Scheiben die Gleitnuten insbesondere entlang ihres äußeren Umfangs auf.

In weiteren beispielhaften Ausführungsformen des Formgebungswerkzeugs können die Aufweitungsdrähte bzw. -elemente in den Gleitnuten radial gesichert bzw. radial fixiert sein. Mit anderen Worten können die Aufweitungsdrähte bzw. -elemente in den Gleitnuten derart geführt sein, dass sie gegen eine Bewegung nach radial außen gesichert sind. Die radiale Fixierung bzw. die radiale Sicherung der Aufweitungsdrähte bzw. -elemente kann beispielhaft eine betätigbare Klemme sein, oder geometrisch durch die Gleitnut selbst ausgebildet sein. Die radiale Sicherung der Aufweitungsdrähte bzw. -elemente ermöglicht vorteilhaft eine besonders genaue Führung der Aufweitungsdrähte bzw. -elemente, so dass ein gezieltes schrittweises Aufweiten auf definierte Durchmesser sichergestellt ist, und im Weiteren ein gezielter Aufweitungswinkel einstellbar ist. Darüber hinaus ermöglicht die radiale Sicherung der Aufweitungsdrähte bzw. -elemente, dass ein lokales Überlagern zweier oder mehrerer Aufweitungsdrähte bzw. -elemente verhindert werden kann, was insbesondere dabei hilft lokal kritische Deformationen bzw. Dehnungen am Formgedächtniswerkstück, während der Formgebung bzw. während dem Aufweiten zu verhindern.

In weiteren beispielhaften Ausführungsformen können die Scheiben entlang ihres äußeren Umfangs und zwischen zwei benachbarten Gleitnuten eine konkave Form bzw. einen radial einwärts gerichteten Rücksprung aufweisen. Die konkave Form bzw. der radial einwärts gerichtete Rücksprung zwischen zwei benachbarten Gleitnuten stellt weiter sicher, dass sich die beweglichen Scheiben und das Formgedächtniswerkstück nicht kontaktieren, wodurch eine axiale Erstreckung des Formgedächtniswerkstücks und/oder eine axiale Anordnung des Formgedächtniswerkstücks an dem Formgebungswerkzeug, während des Aufweitens, beibehalten werden kann.

Darüber hinaus ermöglichen die konkave Form bzw. die radialen Rücksprünge zwischen zwei benachbarten Gleitnuten die Wärmekapazität des Formgebungswerkzeugs weiter vorteilhaft zu verringern.

In bevorzugten Ausführungsformen des Formgebungswerkzeugs zur Formgebung eines Formgedächtniswerkstücks kann das Formgebungswerkzeug ferner:
- eine Erwärmungseinrichtung, bevorzugt ein erhitzbares Salzbad aufweisen; und/oder
- eine Abkühleinrichtung, bevorzugt ein Wasserbad, aufweisen.

Durch die Erwärmungseinrichtung wird vorteilhaft eine Einrichtung zum Erwärmen, insbesondere zum wiederholten bzw. erneuten Erwärmen bzw. zum Weitererwärmen des Formgedächtniswerkstücks bereitgestellt. Die Erwärmungseinrichtung kann beispielsweise ein Bad mit einem Erwärmungsmedium umfassen, wie beispielsweise ein Salzbad. Alternativ oder zusätzlich kann die Erwärmungseinrichtung andere Hochtemperaturflüssigkeiten aufweisen, sowie eine induktive, konvektive, auf elektrischem Widerstand basierende, strahlungsbasierte Erwärmungsmittel, wie Laser oder ähnliches, umfassen, oder eine Kombination davon umfassen.

Durch die Abkühleinrichtung wird vorteilhaft eine Einrichtung zum Abkühlen bereitgestellt. Die Abkühleinrichtung kann beispielhaft eines oder mehrere Gebläse, ein fluidisches Abkühlmedium und/oder ein Wasserbad umfassen. Alternativ oder zusätzlich kann die Abkühleinrichtung einen Gasbehälter mit Gas, insbesondere mit Edelgas wie Stickstoff, Argon, etc. umfassen, welches beispielhaft unter Druck gelagert ist und bei Bedarf hin zum Formgedächtniswerkstück ausströmt.

Während eines oder mehrere Gebläse insbesondere geeignet sind, um gegebenenfalls ein Formgedächtniswerkstück auf eine Zwischentemperatur, unterhalb einer Formgebungstemperatur, zu senken, eignet sich ein Wasserbad insbesondere um ein finales Abkühlen eines Formgedächtniswerkstücks auf eine Temperatur noch unterhalb der Zwischentemperatur abzusenken, und beispielhaft durch Abschrecken auf etwa Raumtemperatur oder eine noch niedrigere Temperatur abzusenken. Ein Gasbehälter, der beispielsweise Edelgas umfasst, kann insbesondere für beide Abkühlvorgänge geeignet sein.

In alternativen Ausführungsformen können die Erwärmungseinrichtung und/oder die Abkühleinrichtung als externe Einrichtungen bereitgestellt werden bzw. an dem Formgebungswerkzeug und/oder in dessen Nähe angeordnet sein.

In weiteren beispielhaften Ausführungsformen des Formgebungswerkzeugs, kann das Formgebungswerkzeug eine Abwurfeinrichtung aufweisen, die konfiguriert ist, das Formgedächtniswerkstück von dem Formgebungswerkzeug abzuwerfen, beispielsweise in eine Abkühleinrichtung abzuwerfen. Die Abwurfeinrichtung kann beispielhaft einen Hebel oder ähnliches umfassen.

In bevorzugten Ausführungsformen wird die Abwurfeinrichtung integral durch das Verfahrrohr gebildet, insbesondere durch das Verfahrrohr und den Aktor, welche konfiguriert sein können das Formgedächtniswerkstück abzuwerfen, indem das Verfahrrohr in der zum Aufweiten entgegengesetzten Richtung bewegt bzw. verfahren wird, wodurch vorteilhaft keine weiteren Abwurfelemente nötig sind.

Ein weiterer Aspekt der Erfindung betrifft ein Verfahren zur Formgebung eines Formgedächtniswerkstücks, ähnlich zum vorgenannten Verfahren zur Formgebung eines Formgedächtniswerkstücks. Das Verfahren zur Formgebung eines Formgedächtniswerkstücks des weiteren Aspekts umfasst:
- Bereitstellen eines Formgedächtniswerkstücks aufweisend einen ersten Durchmesser und eine vorbestimmte Formgebungstemperatur;
- Anordnen des Formgedächtniswerkstücks an einem Formgebungswerkzeug;
- Erwärmen des Formgedächtniswerkstücks auf die Formgebungstemperatur;
- Aufweiten des Formgedächtniswerkstücks auf einen zweiten Durchmesser, der größer ist als der erste Durchmesser;
- Abwerfen des Formgedächtniswerkstücks von dem Formgebungswerkzeug; und
- finales Abkühlen des Formgedächtniswerkstücks auf eine Abkühltemperatur.

Das Verfahren zur Formgebung eines Formgedächtniswerkstücks des weiteren Aspekts weist dabei, insbesondere im Vergleich zum zuvor genannten Verfahren zur Formgebung eines Formgedächtniswerkstücks, nur einen Schritt des Erwärmens auf die Formgebungstemperatur, nur einen Schritt des Aufweitens des Formgedächtniswerkstücks, und insbesondere keinen Schritt des Änderns der Temperatur des Formgedächtniswerkstücks auf eine Zwischentemperatur auf, insbesondere also keinen Schritt des Abkühlens oder Weitererwärmens auf eine Zwischentemperatur.

Das Verfahren des weiteren Aspekts ermöglicht dabei vorteilhaft ein besonders zeit- und energieeffizientes Verfahren zur Formgebung eines Formgedächtniswerkstücks bereitzustellen, insbesondere da das Formgedächtniswerkstück von dem Formgebungswerkzeug abgeworfen wird, um das Formgedächtniswerkstück auf die Abkühltemperatur abzukühlen. Somit wird ein übermäßiges Abkühlen des Formgebungswerkzeugs verhindert, so dass in einem darauffolgenden Verfahren zur Formgebung eines Formgedächtniswerkstücks ein vorteilhaft verringerter Energieverbrauch ermöglicht wird, in dem das Formgebungswerkzeug gegenüber einem weiteren zu formenden Formgedächtniswerkstück bereits erwärmt ist, so dass insbesondere auch ein Erwärmen des Formgedächtniswerkstücks sodann schneller erfolgen kann. Weiterhin ermöglicht das Abwerfen des Formgedächtniswerkstücks von dem Formgebungswerkzeug, dass das Formgedächtniswerkstück einer genau definierten Abkühlrate unterworfen werden kann, da dem Formgedächtniswerkstück kein thermisch träges Formgebungswerkzeug anhaftet, was wiederum die Genauigkeit der Einstellung der Produkteigenschaften des Formgedächtniswerkstücks insbesondere bzgl. Geometrie, Austenit-finish-Temperatur, sowie der auftretenden Kräfte bei Verformung verbessert. Schließlich verhindert das Abwerfen des Formgedächtniswerkstücks auch, dass ein separater Schritt zur Trennung des Formgedächtniswerkstücks von dem Formgebungswerkzeug vorgenommen werden muss, was die Prozessökonomie weiter verbessert, und darüber hinaus die Automation des Verfahrens ermöglicht.

Die zum vorhergehenden Verfahren sowie zum vorhergehenden Formgebungswerkzeug bevorzugten, beispielhaften und alternativen Ausführungsformen beziehen sich gleichermaßen, samt ihrer Vorteile, auch auf das Verfahren des weiteren Aspekts.

Genauso beziehen sich die zum Verfahren des ersten und des weiteren Aspekts bevorzugten, beispielhaften und alternativen Ausführungsformen gleichermaßen, samt ihrer Vorteile, auf das Formgebungswerkzeug, und umgekehrt.

Im Folgenden werden Ausführungsformen der Erfindung anhand der beiliegenden Figuren näher beschrieben. Es versteht sich, dass die vorliegende Erfindung nicht auf diese Ausführungsformen beschränkt ist, und dass einzelne Merkmale der Ausführungsformen im Rahmen der beiliegenden Ansprüche zu weiteren Ausführungsformen kombiniert werden können.

Es zeigt:
- Figur 1: ein Flussdiagramm zum Verfahren zur Formgebung eines Formgedächtniswerkstücks;
- Figur 2: eine Skizze eines Abschnitts eines Formgebungswerkzeugs;
- Figur 3: eine Skizze eines Abschnitts einer Scheibe eines Formgebungswerkzeugs;
- Figur 4a: eine abschnittsweise Darstellung eines Formgebungswerkzeugs in einem ersten Zustand;
- Figur 4b: eine abschnittsweise Darstellung eines Formgebungswerkzeugs in einem zweiten Zustand;
- Figur 4c: eine abschnittsweise Darstellung eines Formgebungswerkzeugs in einem dritten Zustand; und
- Figur 5: einen skizzierten Temperatur-Zeit-Verlauf gemäß eines Beispiels des vorliegenden Verfahrens.

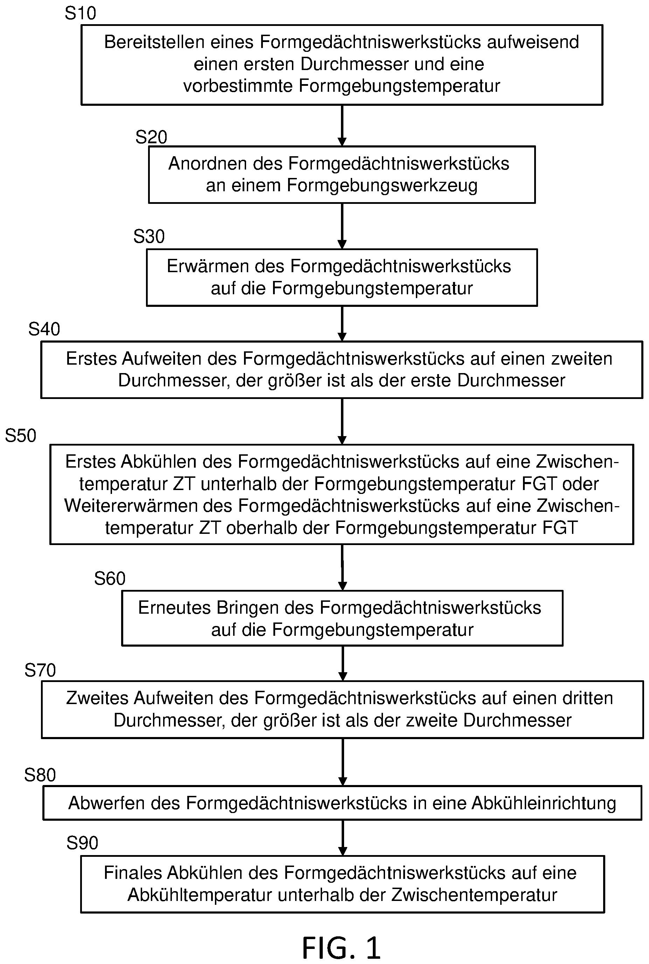

**Fig.** 1 zeigt ein Flussdiagramm eines beispielhaften Verfahrens, umfassend die Schritte:

| | |
|---|---|
| S10: | Bereitstellen eines Formgedächtniswerkstücks 100 aufweisend einen ersten Durchmesser und eine vorbestimmte Formgebungstemperatur FGT; |
| S20: | Anordnen des Formgedächtniswerkstücks 100 an einem Formgebungswerkzeug 10; |
| S30: | Erwärmen des Formgedächtniswerkstücks 100 auf die Formgebungstemperatur FGT; |
| S40: | Erstes Aufweiten des Formgedächtniswerkstücks 100 auf einen zweiten Durchmesser, der größer ist als der erste Durchmesser; |
| S50: | Erstes Abkühlen des Formgedächtniswerkstücks 100 auf eine Zwischentemperatur ZT unterhalb der Formgebungstemperatur FGT oder Weitererwärmen des Formgedächtniswerkstücks 100 auf eine Zwischentemperatur ZT oberhalb der Formgebungstemperatur FGT; |
| S60: | Erneutes Bringen des Formgedächtniswerkstücks 100 auf die Formgebungstemperatur FGT; |
| S70: | Zweites Aufweiten des Formgedächtniswerkstücks 100 auf einen dritten Durchmesser, der größer ist als der zweite Durchmesser; |
| S80: | Abwerfen des Formgedächtniswerkstücks 100 von dem Formgebungswerkzeug 10; und |
| S90: | Finales Abkühlen des Formgedächtniswerkstücks 100 auf eine Abkühltemperatur unterhalb der Zwischentemperatur ZT. |

Das Verfahren nach Fig. 1 gibt ein beispielhaftes Verfahren zur Formgebung eines Formgedächtniswerkstücks 100 wieder. Durch die mehreren Schritte des Aufweitens S40 und S70, wobei jeweils bei einer Formgebungstemperatur FGT des Formgedächtniswerkstücks 100 das Formgedächtniswerkstück 100 aufgeweitet wird, und den dazwischen liegenden Schritt des Abkühlens oder Weitererwärmens auf eine Temperatur unterhalb bzw. oberhalb der Formgebungstemperatur FGT, wird die Gesamtverformung des Formgedächtniswerkstücks 100, um im Wesentlichen eine Zielform bzw. einen Zieldurchmesser zu erhalten, vorteilhaft aufgeteilt. Durch das Aufteilen der Verformung auf mehrere Schritte des Aufweitens können vorteilhaft selbst große Durchmesseränderungen ausgehend von einem ersten bzw. initialen Durchmesser hin zu einem Zieldurchmesser bzw. einem finalen Durchmesser realisiert werden, wobei Schäden am Formgedächtniswerkstück 100 vorteilhaft verringert oder verhindert werden.

Gleichzeitig wird durch das Abwerfen des Formgedächtniswerkstücks 100 im Schritt S80, nach dem Aufweiten auf den Zieldurchmesser bzw. den dritten Durchmesser im Sinne des Schrittes S70, ermöglicht, dass sich das finale Abkühlen im Schritt S90 gezielt auf das Formgedächtniswerkstück 100 richten kann, wodurch ein energetisch aufwendiges Abkühlen und ggf. Wiedererwärmen des Formgebungswerkzeugs 10 verhindert werden kann. Ferner werden hierdurch thermische Beanspruchungen des Formgebungswerkzeugs 10 vorteilhaft reduziert.

Darüber hinaus können die Schritte S30 und S60 des Erwärmens bzw. Bringens des Formgedächtniswerkstücks 100 auf die Formgebungstemperatur FGT, insbesondere jeweils das Erwärmen bzw. Bringen des Formgedächtniswerkstücks 100 auf die Formgebungstemperatur FGT und das Erwärmen bzw. Bringen des Formgebungswerkzeugs 10 auf die Formgebungstemperatur FGT umfassen. Weiterhin können in beispielhaften Ausführungsformen entweder das Formgebungswerkzeug 10 oder das Formgedächtniswerkstück 100 mittelbar durch das Erwärmen oder Abkühlen des jeweils anderen von dem Formgebungswerkzeug 10 und dem Formgedächtniswerkstück 100 erwärmt bzw. abgekühlt werden.

Das Verfahren zur Formgebung eines Formgedächtniswerkstücks 100 ist nicht auf die Schritte nach Fig. 1 begrenzt. Vielmehr können zum Erreichen eines bestimmten Zieldurchmessers bzw. finalen Durchmessers ein oder mehrere weitere Schritte des Abkühlens oder Weitererwärmens auf eine Zwischentemperatur ZT unterhalb bzw. oberhalb der Formgebungstemperatur FGT, erneutes Bringen des Formgedächtniswerkstücks 100 auf die Formgebungstemperatur FGT, und Aufweiten des Formgedächtniswerkstücks 100 auf einen Durchmesser, der gegenüber dem vorhergehenden Schritt des Aufweitens vergrößert ist, angelehnt an die jeweiligen Schritte S50, S60 und S70 nach Fig. 1, vorgenommen werden. Bevorzugt derart, dass mit dem letzten Schritt des Aufweitens des Formgedächtniswerkstücks 100 eine Zielform bzw. ein Zieldurchmesser des Formgedächtniswerkstücks 100 erreicht wird, und so dass als nächstes die Schritte S80 und S90 folgen können. Der Zieldurchmesser des Formgedächtniswerkstücks 100 kann dabei ein Durchmesser sein, welcher größer ist als ein vorbestimmter Durchmesser, den das Formgedächtniswerkstück 100 konfiguriert ist im Einsatz, beispielsweise nach Einbringen in einen menschlichen Körper, einzunehmen, um ein mögliches Rückfedern des Formgedächtniswerkstücks 100 beim Abkühlen nach dem Abwerfen von dem Formgebungswerkzeug 10 zu kompensieren.

In alternativen Ausführungsformen kann das Verfahren auf die Schritte S10, S20, S30, S40, S80 und S90 beschränkt sein, so dass das Formgedächtniswerkstück 100 mittels eines einzelnen Schritts ausgehend von einem ersten bzw. initialen Durchmesser hin zu einem Zieldurchmesser bzw. finalen Durchmesser aufgeweitet wird. Ein solches Verfahren stellt ein besonders schnelles und energieeffizientes Verfahren zur Formgebung eines Formgedächtniswerkstücks 100 bereit, welches gleichzeitig durch eine genau definierbare Abkühlrate eine genaue Einstellung der Produkteigenschaften des Formgedächtniswerkstücks, wie insbesondere Geometrie und Austenit-finish-Temperatur, ermöglicht.

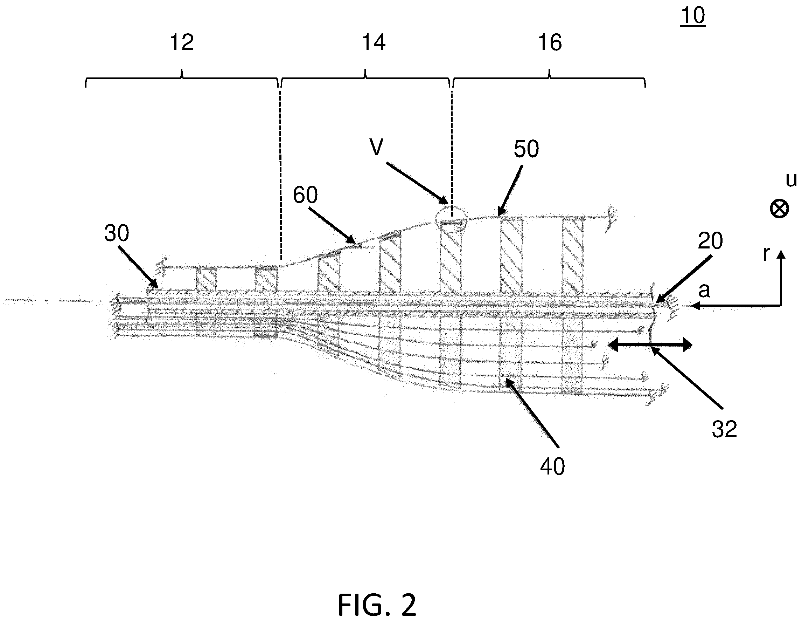

**Fig.** 2 zeigt eine Skizze eines Abschnitts eines Formgebungswerkzeugs 10. Ebenfalls in Fig. 2 ist ein Koordinatensystem wiedergegeben, welches insbesondere die axiale Richtung a, die radiale Richtung r und die Umfangsrichtung u aufweist. In der Darstellung der Fig. 2 erstreckt sich die axiale Richtung a im Wesentlichen von rechts nach links, die radiale Richtung r im Wesentlichen von der axialen Richtung a aus radial nach außen, beispielhaft dargestellt als von unten nach oben, und die Umfangsrichtung u entlang eines Umfangs eines bevorzugt kreisförmigen Elements des Formgebungswerkzeugs 10, beispielhaft dargestellt als in die Zeichenebene hineinzeigend.

Das Formgebungswerkzeug 10, wie in Fig. 2 dargestellt, weist insbesondere ein Führungselement 20 sowie ein Verfahrrohr 30, welches verfahrbar an dem Führungselement 20 angeordnet ist, auf. Das Verfahrrohr 30 kann dabei zumindest teilweise gleitend an dem Führungselement 20 abgestützt sein. Wie in Fig. 2 gezeigt, kann sich das Führungselement 20 im Wesentlichen in axialer Richtung a erstrecken und beispielsweise durch einen Stab oder Hohlzylinder gebildet sein.

Das Verfahrrohr 30 erstreckt sich bevorzugt zumindest abschnittsweise parallel zum Führungselement 20. Mit anderen Worten kann sich das Verfahrrohr 30 zumindest abschnittsweise entlang der axialen Richtung a erstrecken. Das Verfahrrohr 30 kann insbesondere hohl, bevorzugt hohlzylindrisch, ausgebildet sein, und das Führungselement 20 zumindest abschnittsweise in sich aufnehmen.

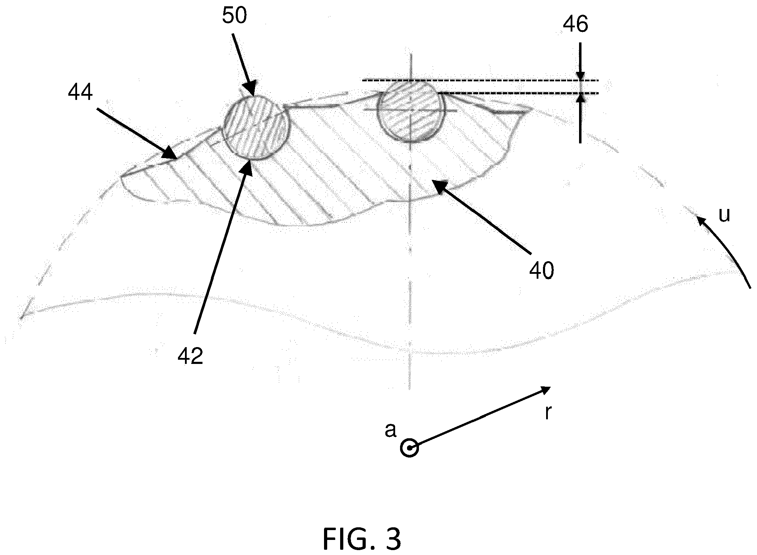

Wie weiterhin in Fig. 2 gezeigt, sind an dem Verfahrrohr 30 mehrere Scheiben 40 angeordnet, und Aufweitungsdrähte bzw. (z.B. stegartige, flexible) Aufweitungselemente 50 derart angeordnet bzw. angebracht, dass sie zwischen den Scheiben 40 gespannt sind bzw. sich zwischen den Scheiben 40 erstrecken. Wie in Fig. 3 weiter verdeutlicht, verlaufen die Aufweitungsdrähte bzw. -elemente 50 bevorzugt entlang eines Außenumfangs der Scheiben 40 bzw. durch Gleitnuten 42 der Scheiben 40.

Die Scheiben 40 sind bevorzugt entlang des Verfahrrohrs 30 in vorbestimmten axialen Abständen, also Abständen im Wesentlichen entlang der axialen Richtung a, zueinander angeordnet. Die Scheiben 40 können dabei im Wesentlichen äquidistant zueinander angeordnet sein oder einen zueinander variierenden Abstand aufweisen. Das Formgebungswerkzeug 10 kann dabei eine beliebige Anzahl an Scheiben 40 aufweisen, bzw. es kann eine beliebige Anzahl an Scheiben 40 an dem Verfahrrohr 30 angeordnet sein.

Die Anzahl der Scheiben 40, welche an dem Verfahrrohr 30 angeordnet ist, kann mindestens der Anzahl der verschiedenen Durchmesser entsprechen, welche ein zu formendes Formgedächtniswerkstück 100 schrittweise im Verfahren zur Formgebung eines Formgedächtniswerkstücks 100 einnimmt.

Darüber hinaus kann in einem Übergangsbereich 14 zwischen zwei diskreten Durchmesserbereichen 12, 16, die jeweils durch eine Mehrzahl von Scheiben 40 mit gleichem Durchmesser gebildet sind, keine Scheibe 40 oder eine beliebige Anzahl von Scheiben 40 an dem Verfahrrohr 30 angeordnet sein, um den Übergangsbereich 14 zu stützen und einen definierten Aufweitungswinkel 60, auch während der Formung eines Formgedächtniswerkstücks 100, sicherzustellen.

Der Aufweitungswinkel 60 stellt sich als Winkel im Übergangsbereich 14 des Formgebungswerkzeugs 10 ein, insbesondere als Winkel dem Aufweitungsdraht 50 folgend gegenüber der axialen Richtung a. Der Aufweitungswinkel 60 wird bestimmt durch den Durchmesser zweier aufeinanderfolgender Scheiben 40 und den axialen Abstand zwischen den betreffenden zwei aufeinanderfolgenden Scheiben 40. Der Aufweitungswinkel 60 beschreibt dabei, in Zusammenwirkung mit einer Verfahrgeschwindigkeit des Verfahrrohrs 30, die Verformung pro Zeit bzw. die Durchmesseränderung pro Zeit, und damit die Kraft, welche auf das Formgedächtniswerkstück 100 aufgebracht wird.

Damit beeinflusst der Aufweitungswinkel 60 die Prozesszeit, und darüber hinaus ganz wesentlich die Geometrie des Formgebungswerkzeugs 10, da je größer der Aufweitungswinkel 60 ist, ein umso kürzeres Formgebungswerkzeug 10 realisiert werden kann, welches wiederum dazu beiträgt den Materialeinsatz für das Formgebungswerkzeug 10 zu verringern, was dessen Wärmekapazität reduziert und die Energieeffizienz im Verfahren erhöht. Weiterhin ermöglicht ein umso kürzeres Formgebungswerkzeug 10, mit einer dementsprechend vergleichsweise geringen Wärmekapazität, dass eine Erwärmungseinrichtung kompakt ausgeführt sein kann, und dadurch wiederum eine vergleichsweise geringe Verlustleistung aufweist, was wiederum schließlich die Energieeffizienz im Verfahren sowie auch die Energieeffizienz des Formgebungswerkzeugs 10 erhöht. Auf der anderen Seite bewirkt ein großer Aufweitungswinkel 60 ein vergleichsweise leichteres Abrutschen des Formgedächtniswerkstücks 100 an dem Formgebungswerkzeug 10, da mit zunehmendem Aufweitungswinkel 60 die Haftreibung zwischen dem Formgedächtniswerkstück 100 und den Aufweitungsdrähten bzw. -elementen 50 während des Aufweitens abnimmt.

Wie in Fig. 2 mittels der geschweiften Klammern skizziert, können sich im Wesentlichen insbesondere drei verschiedene charakteristische Abschnitte an dem Formgebungswerkzeug 10 ausbilden. Dabei kann sich ein Aufnahmebereich 12 insbesondere an einem axialen ersten Abschnitt des Formgebungswerkzeugs 10 ausbilden, an dem Scheiben 40 in Zusammenwirkung mit daran angeordneten bzw. gespannten Aufweitungsdrähten bzw. -elementen 50 einen ersten diskreten Durchmesser bilden, der geeignet ist, dass Formgedächtniswerkstücke 100 mit einem ersten bzw. initialen Durchmesser daran angeordnet werden, insbesondere unter Vorspannung daran angeordnet zu werden. Der erste bzw. initiale Durchmesser des Formgedächtniswerkstücks 100, und damit der erste diskrete Durchmesser, den die Scheiben 40 mit den daran angeordneten Aufweitungsdrähten 50 im Aufnahmebereich 12 bilden, kann, beispielhaft für Stents, in einem Bereich von etwa 3 mm bis etwa 15 mm liegen, bevorzugt in einem Bereich von etwa 6 mm bis etwa 12 mm liegen. Jedoch sind weder Stents noch andere beispielhafte Formgedächtniswerkstücke, wie Herzklappen, auf die vorgenannten Durchmesser beschränkt. An den Aufnahmebereich 12 angrenzend und in Gegenrichtung der axialen Richtung a folgend, kann sich beispielhaft ein Übergangsbereich 14 an dem Formgebungswerkzeug 10 erstrecken, der beispielhaft einen Übergang zwischen dem ersten und einem zweiten diskreten Durchmesser bildet. Im Übergangsbereich 14 muss nicht zwingend eine Scheibe 40 angeordnet sein. Jedoch können eine oder mehrere Scheiben 40, welche im Übergangsbereich 14 angeordnet sind, das Aufweiten eines Formgedächtniswerkstücks 100 mit einem gezielten Aufweitungswinkel 60 unterstützen, wodurch ein besonders zuverlässiges Verfahren zur Formgebung eines Formgedächtniswerkstücks 100 bereitgestellt werden kann. An den Übergangsbereich 14 angrenzend und in Gegenrichtung der axialen Richtung a folgend, kann sich beispielhaft ein diskreter Durchmesserbereich 16 erstrecken, innerhalb dessen zumindest zwei Scheiben 40 mit insbesondere dem gleichen Durchmesser angeordnet sein können.

Die vorstehend bezeichnete Gegenrichtung der axialen Richtung a ist eine Richtung die im Wesentlichen parallel entgegengesetzt zur axialen Richtung a verläuft bzw. zeigt.

Die Scheiben 40 im diskreten Durchmesserbereich 16 können insbesondere einen gegenüber den Scheiben 40 im Aufnahmebereich 12 größeren Durchmesser aufweisen. Die Scheiben 40, die im diskreten Durchmesserbereich 16 angeordnet sind können mit den daran angeordneten bzw. gespannten Aufweitungsdrähten 50 einen zweiten diskreten Durchmesser bilden, um ein Formgedächtniswerkstück 100 auf einen entsprechenden Durchmesser aufzuweiten.

Das Verhältnis des mittleren Durchmessers des Übergangsbereichs 14 zum Durchmesser der Scheiben im Aufnahmebereich 12 kann bevorzugt im Bereich von etwa 1,5 bis etwa 1,9 liegen, besonders bevorzugt bei etwa 1,85 liegen. Weiter kann das Verhältnis des Durchmessers der Scheiben 40 im diskreten Durchmesserbereich 16 zum mittleren Durchmesser des Übergangsbereichs 14 bevorzugt im Bereich von etwa 1,5 bis etwa 1,9 liegen, besonders bevorzugt bei etwa 1,85 liegen.

In alternativen Ausführungsformen kann das Verhältnis des Durchmessers der Scheiben im diskreten Durchmesserbereich 16 zum Durchmesser der Scheiben im Aufnahmebereich 12 bevorzugt im Bereich von etwa 1,5 bis etwa 1,9 liegen, besonders bevorzugt bei etwa 1,85 liegen.

In Fig. 2 lediglich skizziert ist ein Aktor 32 angedeutet, welcher insbesondere konfiguriert ist die Scheiben 40 in und entgegen der im Wesentlichen axialen Richtung a bzw. parallel zum Verfahrrohr 30 zu verschieben bzw. zu bewegen. Dazu können einerseits die Scheiben 40 beweglich bzw. verfahrbar an dem Verfahrrohr 30 angeordnet sein und direkt durch den Aktor 32 bewegbar sein.

Andererseits können die Scheiben 40 an dem Verfahrrohr 30 fixiert sein bzw. fest angeordnet sein und konfiguriert sein, durch den Aktor 32 mittelbar, beispielsweise über das Verfahrrohr 30, bewegt bzw. verschoben zu werden. Die Bewegung bzw. das Verschieben durch den Aktor 32 ist bevorzugt als Bewegung bzw. Verschieben im Wesentlichen entlang der axialen Richtung a bzw. parallel zum Verfahrrohr 30 konfiguriert.

In weiteren beispielhaften Ausführungsformen kann eine Scheibe 40 mit einer Ausformung versehen sein, um beim Passieren des Formgedächtniswerkstücks 100 eine spezifische Umformung dessen zu bewirken, wie beispielsweise die Ausbildung eines Hakens an dem Formgedächtniswerkstück 100.

In Fig. 2 angedeutet, ist weiterhin eine Stelle die als Vergrößerung V markiert ist. Diese Stelle der Vergrößerung V stellt einen beispielhaften Ausschnitt einer Scheibe 40 genauer dar. Die Vergrößerung V ist in Fig. 3 weiter erläutert.

**Fig. 3** zeigt eine Skizze eines Abschnitts einer Scheibe 40 eines beispielhaften Formgebungswerkzeugs 10. Die Skizze in Fig. 3 ist dabei als teilweise Schnittdarstellung einer Scheibe 40 wiedergegeben, wobei neben der Scheibe 40 auch mehrere Aufweitungsdrähte bzw. -elemente 50 zur Veranschaulichung geschnitten mit dargestellt sind. Weiterhin sind in Fig. 3 schematisch die axiale Richtung a, aus dem Zeichnungsblatt herauszeigend, die radiale Richtung r, welche ausgehend von der axialen Richtung a nach radial auswärts zeigt, sowie die Umfangsrichtung u, welche in Umfangsrichtung u der zumindest abschnittsweise kreisförmigen Scheibe 40 verläuft.

Wie in Fig. 3 gezeigt, kann jeder der Aufweitungsdrähte bzw. -elemente 50 in zumindest einer Gleitnut 42 angeordnet sein, wobei die Gleitnut 42 bevorzugt an einer Stelle entlang eines äußeren Umfangs bzw. entlang einer Außenumfangsseite der Scheibe 40 angeordnet ist. Die Gleitnut 42 bzw. die Gleitnuten 42 können insbesondere eine Öffnung aufweisen, welche in radialer Richtung r nach außen offen ist, so dass ein in der Gleitnut 42 angeordneter Aufweitungsdraht bzw. -element 50 in radialer Richtung r gegenüber der Scheibe 40, insbesondere gegenüber einem äußeren Umfang der Scheibe 40 bzw. einer Außenumfangsseite der Scheibe 40 hervorsteht.

Das Hervorstehen des Aufweitungsdrahts bzw. -elements 50 gegenüber der Scheibe 40 kann insbesondere mittels des Überstands 46 verdeutlicht werden. Der Überstand 46 beschreibt in der Schnittbetrachtung nach Fig. 3 einen Abstand zwischen der in radialer Richtung r äußersten Stelle des Aufweitungsdrahts bzw. -elements 50 gegenüber einer in radialer Richtung r äußersten Stelle der Scheibe 40, welche an die Gleitnut 42 angrenzt. In beispielhaften Ausführungsformen kann der Überstand 46 im Verhältnis zum Durchmesser bzw. Querschnitt des Aufweitungsdrahts bzw. -elements 50 etwa 5 % bis etwa 60 % betragen, bevorzugt etwa 8 % bis etwa 40 % betragen, besonders bevorzugt etwa 10 % bis etwa 20 % betragen. Vorteilhaft ermöglicht eine Gleitnut 42, welche derart ausgebildet ist, dass sie mit dem darin angeordneten Aufweitungsdraht bzw. -element 50 den Überstand 46 ausgebildet, der im Verhältnis zum Durchmesser des Aufweitungsdrahts 50 weniger als 50 % beträgt, dass der Aufweitungsdraht bzw. -element 50 in der Gleitnut 42 radial gesichert bzw. radial fixiert ist.

In beispielhaften Ausführungsformen kann die Gleitnut 42 der Scheibe 40 derart ausgebildet sein, dass ein darin zumindest abschnittsweise angeordneter Aufweitungsdraht bzw. -element 50 gleitend geführt werden kann. Mit anderen Worten kann die Gleitnut 42 der Scheibe 40 derart ausgebildet sein, dass die Scheibe 40 relativ zum Aufweitungsdraht 50 verschiebbar ist. Eine Gleitnut 42 kann dazu in beispielhaften Ausführungsformen in axialer Richtung a insbesondere als Bohrung ausgebildet sein, welche einen Durchmesser bzw. Querschnitt aufweist, der mindestens dem Durchmesser bzw. Querschnitt des darin zumindest abschnittsweise angeordneten Aufweitungsdrahts bzw. -elements 50 entspricht. Dies ermöglicht vorteilhaft, dass ein Formgedächtniswerkstück 100 derart an einem Formgebungswerkzeug 10 anordenbar ist, dass das Formgedächtniswerkstück 100 von den Aufweitungsdrähten bzw. -elementen 50 gehalten wird, und gleichzeitig von den Scheiben 40 passierbar ist, beispielsweise in axialer Richtung a bzw. in einer zum Verfahrrohr 30 parallelen Richtung.

In bevorzugten Ausführungsformen entspricht die Anzahl der Gleitnuten 42 mindestens der Anzahl der an den Scheiben 40 anzuordnenden Aufweitungsdrähte bzw. -elemente 50, so dass jeder Aufweitungsdraht bzw. -element 50 in einer separaten Gleitnut 42 anordenbar ist.

In weiter bevorzugten Ausführungsformen sind die Gleitnuten 42 im Wesentlichen äquidistant am äußeren Umfang der Scheibe 40 bzw. an einer Außenumfangsseite der Scheibe 40 angeordnet, so dass das Formgebungswerkzeug 10 ein insbesondere gleichmäßiges Aufweiten insbesondere runder bzw. im Wesentlichen runder Werkstücke ermöglicht. In alternativen Ausführungsformen kann die Anordnung der Gleitnuten 42 auch von einer äquidistanten Anordnung an der Scheibe 40 abweichen. Weiterhin können die Gleitnuten 42 in alternativen Ausführungsformen in einem Scheibeninneren, also nicht am Außenumfang der Scheibe 40, angeordnet sein.

Wie weiterhin in Fig. 3 gezeigt kann die Scheibe 40 entlang ihres äußeren Umfangs bzw. entlang ihrer Außenumfangsseite und zwischen zwei benachbarten Gleitnuten 42 einen konkaven Abschnitt 44 bzw. einen radial einwärts gerichteten Rücksprung aufweisen.

Der konkave Abschnitt 44 bzw. der radial einwärts gerichtete Rücksprung zwischen zwei benachbarten Gleitnuten 42 stellt vorteilhaft sicher, dass bei einer im Wesentlichen in axialer Richtung a verlaufenden Relativbewegung der Scheiben 40 relativ zu den Aufweitungsdrähten 50 eine Kontaktierung eines an den Aufweitungsdrähten 50 angeordneten Formgedächtniswerkstücks 100 und der Scheiben 40 verhindert werden kann. Dies ermöglicht weiterhin die axiale Erstreckung des Formgedächtniswerkstücks 100 während des Aufweitens vorteilhaft beizubehalten, und zusätzlich bei hohen Temperaturen eine Verunreinigung durch Kontakt zwischen dem Formgedächtniswerkstück 100 und den Scheiben 40 zu verhindern.

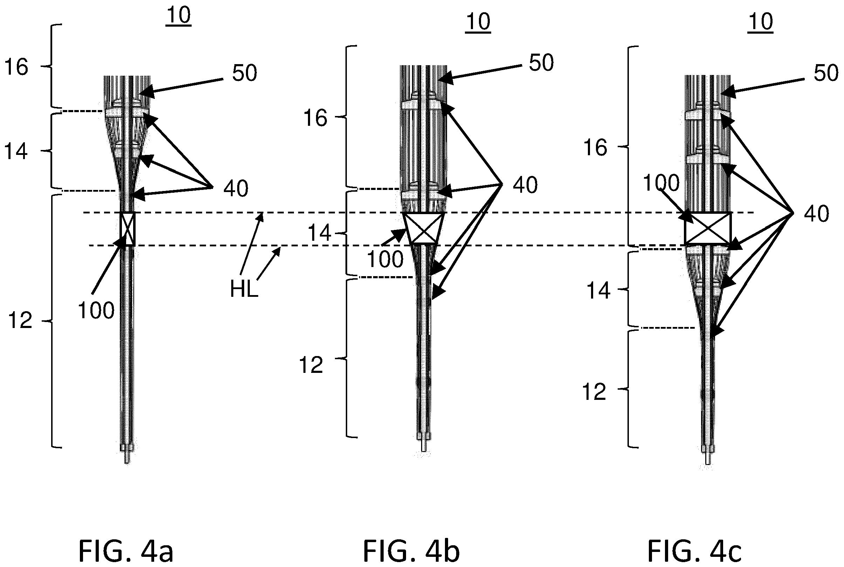

Die **Figuren 4a, 4b und 4c** zeigen einen Abschnitt eines beispielhaften Formgebungswerkzeugs 10 in einem ersten Zustand (Fig. 4a), einem zweiten Zustand (Fig. 4b) und in einem dritten Zustand (Fig. 4c). Grundsätzlich zeigen die Figuren 4a, 4b und 4c dabei, welche Stellungen die Scheiben 40 sowie die Vielzahl von Aufweitungsdrähten bzw. -elementen 50 in verschiedenen Schritten eines Verfahrens zur Formgebung eines Formgedächtniswerkstücks 100 einnehmen können. Ein schematisch skizziertes Formgedächtniswerkstück 100 ist beispielhaft an dem Formgebungswerkzeug 10 angeordnet dargestellt.

Wie in den Figuren 4a, 4b und 4c angedeutet, können die Aufweitungsdrähte bzw. -elemente 50 insbesondere an einem unteren Fixierabschnitt des Formgebungswerkzeugs 10 fixiert sein. Bevorzugt können insbesondere die distalen Enden der Aufweitungsdrähte bzw. -elemente 50 am unteren Fixierabschnitt des Formgebungswerkzeugs 10 fixiert sein. In beispielhaften Ausführungsformen, wie jedoch nicht in den Figuren gezeigt, können sich die Aufweitungsdrähte bzw. -elemente 50 insbesondere zwischen einem oberen Fixierabschnitt und einem unteren Fixierabschnitt des Formgebungswerkzeugs 10 erstrecken und bevorzugt zwischen dem oberen Fixierabschnitt und dem unteren Fixierabschnitt gespannt sein.

Der obere und der untere Fixierabschnitt können beispielsweise an dem Führungselement 20 ausgebildet sein, sind jedoch nicht darauf beschränkt.

In weiteren beispielhaften Ausführungsformen, wie jedoch nicht in den Figuren gezeigt, können die Aufweitungsdrähte bzw. -elemente 50 insbesondere an einem unteren Ende des Führungselements 20 zurückgeführt werden, insbesondere hin zu einem oberen Fixierabschnitt des Formgebungswerkzeugs 10 zurückgeführt werden.

In bevorzugten Ausführungsformen, wie jedoch nicht in den Figuren gezeigt, können jeweils ein erstes Ende und ein zweites Ende der Aufweitungsdrähte bzw. -elemente 50, insbesondere also alle bzw. beide Enden der Aufweitungsdrähte bzw. -elemente 50 an einem oberen Fixierabschnitt bzw. an einem oberen Ende des Formgebungswerkzeugs 10 fixiert sein, wobei die Aufweitungsdrähte bzw. -elemente 50 bevorzugt an einem unteren Ende des Führungselements 20 zurückgeführt werden. Dies ermöglicht vorteilhaft, dass am unteren Ende des Formgebungswerkzeugs 10 keinerlei Fixierung, insbesondere keinerlei mechanische Fixierung der Aufweitungsdrähte bzw. -elemente 50 benötigt wird. Daraus resultiert weiter vorteilhaft, dass das Formgebungswerkzeug 10 auch zur Anbringung besonders kleiner Formgedächtniswerkstücke 100 geeignet ist, da das Anbringen des Formgedächtniswerkstücks 100, insbesondere an einem unteren Abschnitt des Formgebungswerkzeugs 10, insbesondere an dem Aufnahmebereich 12, nicht durch eine Fixierung der Aufweitungsdrähte bzw. -elemente 50 beeinträchtigt wird. Noch weiter vorteilhaft, wird dadurch die Langlebigkeit und Wartung des Formgebungswerkzeugs 10 verbessert, da die Fixierung der Aufweitungsdrähte bzw. -elemente 50 nicht notwendigerweise der direkten Erwärmung durch eine Erwärmungseinrichtung ausgesetzt ist, wie beispielsweise einem Salzbad.

Fig. 4a zeigt einen ersten Zustand des Formgebungswerkzeugs 10, der beispielhaft einen Zustand des Formgebungswerkzeugs 10 beschreibt, wenn dieses geeignet ist, dass ein Formgedächtniswerkstück 100 daran angeordnet wird. Hierzu kann das Formgebungswerkzeug 10 einen ausgeprägten Aufnahmebereich 12 aufweisen, der sich beispielsweise an einem in axialer Richtung a unteren Abschnitt des Formgebungswerkzeugs 10 erstreckt, wobei die Aufweitungsdrähte bzw. -elemente 50 einen Durchmesser bilden, der geeignet ist, ein Formgedächtniswerkstück 100 mit einem ersten bzw. initialen Durchmesser daran anzuordnen. Weiterhin kann das Formgebungswerkzeug 10 neben dem Aufnahmebereich 12 einen Übergangsbereich 14 und einen diskreten Durchmesserbereich 16 aufweisen, die sich in dieser Reihenfolge entgegen der axialen Richtung a an den Aufnahmebereich 12 anschließen bzw. davon erstrecken.

Fig. 4b zeigt einen zweiten Zustand des Formgebungswerkzeugs 10, der beispielhaft einen Zustand des Formgebungswerkzeugs 10 beschreibt, wenn das Formgedächtniswerkstück 100 auf einen zweiten Durchmesser aufgeweitet wurde. Dabei bilden die Aufweitungsdrähte bzw. -elemente 50 im Übergangsbereich 14 einen Durchmesser, den das Formgedächtniswerkstück 100 bzw. Abschnitte des Formgedächtniswerkstücks 100 beispielsweise nach einem ersten Aufweiten aufweist bzw. aufweisen.

Fig. 4c zeigt einen dritten Zustand des Formgebungswerkzeugs 10, der beispielhaft einen Zustand des Formgebungswerkzeugs 10 beschreibt, wenn das Formgedächtniswerkstück 100 auf einen dritten Durchmesser aufgeweitet wurde. Dabei bilden die Aufweitungsdrähte bzw. -elemente 50 im diskreten Durchmesserbereich 16 bevorzugt einen Durchmesser, den das Formgedächtniswerkstück 100 nach einem zweiten Aufweiten aufweist.

Für das Aufweiten selbst, und wie in einem Vergleich zwischen den Figuren 4a, 4b und 4c verdeutlicht, werden dabei bevorzugt zumindest die Scheiben 40, welche den Übergangsbereich 14 und den diskreten Durchmesserbereich 16 bilden, in axialer Richtung a nach unten verschoben bzw. bewegt, so dass diese das Formgedächtniswerkstück 100 bevorzugt passieren bzw. den initialen Aufnahmebereich passieren, an dem das Formgedächtniswerkstück 100 ursprünglich angeordnet wurde. Das Formgedächtniswerkstück 100 kann durch das Passieren der Scheiben 40 vorteilhaft gleichmäßig aufgeweitet werden. Wie Weiterhin aus einem Vergleich der Figuren 4a, 4b und 4c verdeutlicht, kann das Formgedächtniswerkstück 100 selbst über einen Schritt des Aufweitens hinweg an der im Wesentlichen gleichen Position am Formgebungswerkzeug 10 angeordnet bzw. gehalten werden, und seine axiale Erstreckung beibehalten, wie in den Figuren 4a, 4b, 4c mittels der strichtlierten Hilfslinien HL hervorgehoben.

Die Figuren 4a, 4b und 4c sind jeweils nur schematische Darstellungen, welche zur Übersichtlichkeit nicht das gesamte Formgebungswerkzeug 10 wiedergeben. Auch das am Formgebungswerkzeug 10 angeordnete Formgedächtniswerkstück 100 ist lediglich schematisch wiedergegeben und insbesondere hinsichtlich der axialen Erstreckung nicht maßstabsgetreu wiedergegeben.

In weiteren beispielhaften jedoch nicht dargestellten Ausführungsformen des Formgebungswerkzeugs 10, kann dieses einen oder mehrere weitere Übergangsbereiche 14 sowie einen oder mehrere weitere diskrete Durchmesserbereiche 16 aufweisen, welche sich entgegen der axialen Richtung a von dem dargestellten diskreten Durchmesserbereich 16 erstrecken.

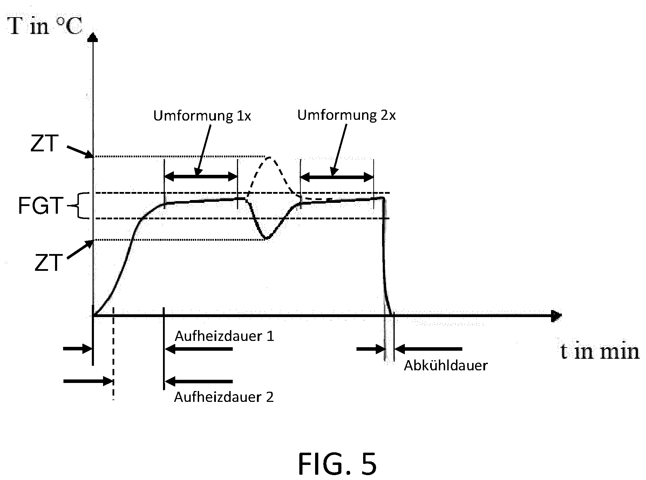

Fig. 5 zeigt einen skizzierten Temperatur-Zeit-Verlauf gemäß eines Beispiels des vorliegenden Verfahrens. Der in Fig. 5 dargestellte Temperatur-Zeit-Verlauf für ein Verfahren zur Formgebung eines Formgedächtniswerkstücks 100 ist als lediglich qualitative Skizze und Übersicht zu bewerten, wie ein Verfahren zur Formgebung eines Formgedächtniswerkstücks 100 ablaufen kann.

Wie in Fig. 5 dargestellt, wird das Formgedächtniswerkstück 100 bevorzugt zunächst auf eine Formgebungstemperatur FGT erwärmt bzw. erhitzt bzw. auf eine Temperatur erwärmt bzw. erhitzt, welche im Bereich der Formgebungstemperatur FGT, für das Formgedächtniswerkstück 100 liegt. Während das Formgedächtniswerkstück 100 sich im Temperaturbereich der Formgebungstemperatur FGT befindet, kann es bevorzugt verformt bzw. umgeformt werden, mit anderen Worten aufgeweitet werden (in Fig. 5 dargestellt mit "Umformung 1x"). Der Umformungsgrad kann dabei beliebig festgelegt werden, liegt jedoch bevorzugt in einem Bereich unterhalb von etwa 1,9, besonders bevorzugt bei etwa 1,85.

Wie in Fig. 5 weiter dargestellt, wird das Formgedächtniswerkstück 100 im Anschluss an ein erstes Umformen bzw. Aufweiten abgekühlt oder weitererwärmt, bevorzugt auf eine Temperatur unterhalb oder oberhalb der Formgebungstemperatur FGT. Diese Temperatur unterhalb bzw. oberhalb der Formgebungstemperatur FGT kann als Zwischentemperatur ZT bezeichnet werden und beispielhaft unterhalb von 500 °C liegen, insbesondere im Bereich von etwa 250 °C bis etwa 500 °C liegen, oder oberhalb von 525 °C liegen, insbesondere im Bereich von etwa 525 °C bis etwa 600 °C.

Anschließend wird das Formgedächtniswerkstück 100 wieder auf die Formgebungstemperatur FGT gebracht, also erwärmt oder abgekühlt, um danach ein weiteres Mal verformt bzw. umgeformt zu werden. Weist das Formgedächtniswerkstück 100 nach dem zweiten Umformen (in Fig. 5 dargestellt mit "Umformung 2x") den gewünschten Zieldurchmesser auf, der dem Formgedächtniswerkstück 100 als Formgedächtnis eingeprägt werden soll, so kann es im Anschluss abgekühlt bzw. abgeschreckt werden (in Fig. 5 veranschaulicht mittels der kurzen "Abkühldauer"), beispielsweise bis auf etwa Raumtemperatur abgekühlt bzw. abgeschreckt werden.

Aus der Kombination aus mehrfacher Umformung des Formgedächtniswerkstücks 100 bei Formgebungstemperatur FGT, zusammen mit der dazwischenliegenden Änderung auf eine Temperatur unterhalb oder oberhalb der Formgebungstemperatur FGT, und dem finalen Abkühlen, sobald das Formgedächtniswerkstück 100 bis zu seiner Zielform bzw. seinem Zieldurchmesser aufgeweitet bzw. umgeformt wurde, werden dem Formgedächtniswerkstück 100 vorbestimmte Formgedächtniseigenschaften bzw. superelastische Eigenschaften, umfassend einen gewünschten Durchmesser, der bei einer vorbestimmten Umgebungstemperatur und einem vorbestimmten Lastzustand eingenommen wird, eingeprägt.

Weiterhin sind in Fig. 5 beispielhaft zwei Aufheizdauern zum Erwärmen des Formgedächtniswerkstücks 100 auf die Formgebungstemperatur FGT dargestellt.

Die "Aufheizdauer 1" stellt dabei den Zeitraum dar, der benötigt wird, wenn das Formgebungswerkzeug 10 nicht vorgewärmt ist, das Verfahren zur Formgebung mittels des Formgebungswerkzeugs 10 also in etwa bei Raumtemperatur gestartet wird. Die "Aufheizdauer 2" stellt demgegenüber einen verkürzten Zeitraum dar, wenn das Formgebungswerkzeug 10 bereits einen formgebenden Zyklus mit einem Formgedächtniswerkstück 100 gefahren hat. Die vergleichsweise verkürzte "Aufheizdauer 2" wird insbesondere dadurch ermöglicht, dass das Formgedächtniswerkstück 100 zum finalen Abkühlen von dem Formgebungswerkzeug 10 abgeworfen wird, wodurch das Formgebungswerkzeug 10 selbst keine erhebliche Abkühlung, im Gegensatz zum abgeworfenen Formgedächtniswerkstück 100, erfährt.

### Bezugszeichenliste

- 10: Formgebungswerkzeug
- 12: Aufnahmebereich
- 14: Übergangsbereich
- 16: diskreter Durchmesserbereich
- 20: Führungselement
- 30: Verfahrrohr
- 32: Aktor
- 40: Scheibe
- 42: Gleitnut
- 44: konkaver Abschnitt
- 46: Überstand
- 50: Aufweitungsdraht
- 60: Aufweitungswinkel
- 100: Formgedächtniswerkstück
- S10-S90: Schritte für ein Verfahren zur Formgebung eines Formgedächtniswerkstücks
- a: axiale Richtung
- r: radiale Richtung
- u: Umfangsrichtung
- FGT: Formgebungstemperatur
- HL: Hilfslinie
- V: Vergrößerung
- ZT: Zwischentemperatur

## Patentansprüche

1. Verfahren zur Formgebung eines Formgedächtniswerkstücks (100), umfassend:
- Bereitstellen eines Formgedächtniswerkstücks (100) aufweisend einen ersten Durchmesser und eine vorbestimmte Formgebungstemperatur (FGT);
- Anordnen des Formgedächtniswerkstücks (100) an einem Formgebungswerkzeug (10);
- Erwärmen des Formgedächtniswerkstücks (100) auf die Formgebungstemperatur (FGT);
- erstes Aufweiten des Formgedächtniswerkstücks (100) auf einen zweiten Durchmesser, der größer ist als der erste Durchmesser;
- erstes Ändern der Temperatur des Formgedächtniswerkstücks (100) auf eine Zwischentemperatur (ZT) unterhalb oder oberhalb der Formgebungstemperatur (FGT);
- erneutes Bringen des Formgedächtniswerkstücks (100) auf die Formgebungstemperatur (FGT);
- zweites Aufweiten des Formgedächtniswerkstücks (100) auf einen dritten Durchmesser, der größer ist als der zweite Durchmesser;
- Abwerfen des Formgedächtniswerkstücks (100) von dem Formgebungswerkzeug (10); und
- finales Abkühlen des Formgedächtniswerkstücks (100) auf eine Abkühltemperatur unterhalb der Zwischentemperatur (ZT).

2. Verfahren zur Formgebung eines Formgedächtniswerkstücks (100) nach Anspruch 1, wobei das erste Ändern der Temperatur des Formgedächtniswerkstücks (100) auf eine Zwischentemperatur (ZT) ein erstes Abkühlen des Formgedächtniswerkstücks (100) auf die Zwischentemperatur (ZT) unterhalb der Formgebungstemperatur (FGT) umfasst, oder ein erstes Weitererwärmen des Formgedächtniswerkstücks (100) auf die Zwischentemperatur (ZT) oberhalb der Formgebungstemperatur (FGT) umfasst,
wobei das erste Ändern der Temperatur des Formgedächtniswerkstücks (100) bevorzugt das Ändern der Temperatur des Formgedächtniswerkstücks (100) um zumindest etwa 25 °C, vorzugsweise um zumindest etwa 40 °C umfasst.

3. Verfahren zur Formgebung eines Formgedächtniswerkstücks (100) nach Anspruch 1 oder 2, wobei
der zweite Durchmesser das etwa 1,5-fache bis etwa 1,9-fache des ersten Durchmessers ist, bevorzugt das etwa 1,85-fache des ersten Durchmessers ist; und/oder
der dritte Durchmesser das etwa 1,5-fache bis etwa 1,9-fache des zweiten Durchmessers ist, bevorzugt das etwa 1,85-fache des zweiten Durchmessers ist.

4. Verfahren zur Formgebung eines Formgedächtniswerkstücks (100) nach einem der Ansprüche 1 bis 3, wobei das erste Aufweiten und das zweite Aufweiten ein Aufweiten des Formgedächtniswerkstücks (100) in radialer Richtung, insbesondere unter Beibehaltung der axialen Erstreckung des Formgedächtniswerkstücks (100), umfasst.

5. Verfahren zur Formgebung eines Formgedächtniswerkstücks (100) nach einem der vorangehenden Ansprüche, wobei während zumindest einem des ersten und zweiten Aufweitens das Formgedächtniswerkstück (100) reibschlüssig gehalten wird.

6. Verfahren zur Formgebung eines Formgedächtniswerkstücks (100) nach Anspruch 5, wobei das Formgedächtniswerkstück (100) während dem zumindest einen des ersten und zweiten Aufweitens des Formgedächtniswerkstücks (100) reibschlüssig an dem Formgebungswerkzeug (10) gehalten wird.

7. Verfahren zur Formgebung eines Formgedächtniswerkstücks (100) nach einem der vorangehenden Ansprüche, wobei das Formgedächtniswerkstück (100):
- eine Nickel-Titan Legierung aufweist; und/oder
- eine runde Form aufweist; und/oder
- ein Stentmuster aufweist.

8. Formgebungswerkzeug (10) zur Formgebung eines Formgedächtniswerkstücks (100), aufweisend:
- ein Führungselement (20), und
- ein Verfahrrohr (30), welches verfahrbar an dem Führungselement (20) angeordnet ist, wobei
-- an dem Verfahrrohr (30) in vorbestimmten axialen Abständen Scheiben (40) angeordnet sind,
-- zwischen den Scheiben (40) Aufweitungsdrähte (50) gespannt sind, und wobei
-- die zwischen den Scheiben (40) gespannten Aufweitungsdrähte (50) Durchmesser bilden, um ein Formgedächtniswerkstück (100) umfänglich daran anzuordnen.

9. Formgebungswerkzeug (10) zur Formgebung eines Formgedächtniswerkstücks (100) nach Anspruch 8, wobei das Formgebungswerkzeug (10) einen Aufnahmebereich (12) zur Anordnung eines Formgedächtniswerkstücks (100) mit einem ersten Durchmesser daran aufweist, und wobei
- das Verfahrrohr (30) konfiguriert ist mittels eines Aktors (32) relativ zum Aufnahmebereich (12) bewegt zu werden, so dass das Formgedächtniswerkstück (100) im Aufnahmebereich (12) von den Scheiben (40) passierbar ist.

10. Formgebungswerkzeug (10) zur Formgebung eines Formgedächtniswerkstücks (100) nach einem der Ansprüche 8 oder 9, wobei die Scheiben (40) derart geformt sind, dass das Formgebungswerkzeug (10) konfiguriert ist mittels einer Bewegung des Verfahrrohrs (30) entlang des Führungselements (20) das Formgedächtniswerkstück (100) schrittweise aufzuweiten.

11. Formgebungswerkzeug (10) zur Formgebung eines Formgedächtniswerkstücks (100) nach einem der Ansprüche 8 bis 10, wobei die Scheiben (40) entlang ihres Umfangs Gleitnuten (42) aufweisen,
wobei entlang jeder Gleitnut (42) ein Aufweitungsdraht (50) durch die jeweilige Scheibe (40) gleitend geführt ist, und
wobei bevorzugt jeder Aufweitungsdraht (50) derart in der jeweiligen Gleitnut (42) angeordnet ist, dass er radial nach außen gegenüber der Scheibe (40), in deren Gleitnut (42) der Aufweitungsdraht (50) geführt ist, vorsteht.

12. Formgebungswerkzeug (10) zur Formgebung eines Formgedächtniswerkstücks (100) nach einem der Ansprüche 8 bis 11, wobei die Aufweitungsdrähte (50) eine Gitterstruktur bilden, insbesondere eine Gitterstruktur zueinander in Umfangsrichtung (u) beabstandeter länglicher Gitterelemente.

13. Formgebungswerkzeug (10) zur Formgebung eines Formgedächtniswerkstücks (100) nach Anspruch 12, wobei die Gitterstruktur abschnittsweise aus einem oder mehreren zylinderförmigen Abschnitten und einem oder mehreren kegelstumpfförmigen Abschnitten gebildet ist,
wobei sich die einen oder mehreren zylinderförmigen Abschnitte mit den einen oder mehreren kegelstumpfförmigen Abschnitten gegenseitig abwechseln.

14. Formgebungswerkzeug (10) nach einem der Ansprüche 8 bis 13, wobei das Verfahrrohr (30) das Führungselement (20) zumindest abschnittsweise in sich aufnimmt.

15. Formgebungswerkzeug (10) zur Formgebung eines Formgedächtniswerkstücks (100) nach einem der Ansprüche 8 bis 14, ferner aufweisend:
- eine Erwärmungseinrichtung, bevorzugt ein erhitzbares Salzbad; und/oder
- eine Abkühleinrichtung, bevorzugt ein Wasserbad.
